# FASCICULE DE BREVET EUROPEEN

(11) **EP 0 488 860 B1**
(45) Date de publication et mention de la délivrance du brevet: **11.10.1995**
(21) Numéro de dépôt: 91403154.7
(22) Date de dépôt: 22.11.1991
(51) Int. Cl.: C07C 45/64, C07C 47/575, C07C 45/61

(54) **Procédé de préparation de composés aromatiques polyalkoxylés**
Verfahren zur Herstellung von polyalkoxylierten aromatischen Verbindungen
Method for the production of polyalkoxylated aromatic compounds

(30) Priorité: 30.11.1990 FR 9015001
(43) Date de publication de la demande: 03.06.1992
(73) Titulaire: RHONE-POULENC CHIMIE, 92408 Courbevoie Cédex (FR)
(72) Inventeur: Nobel, Dominique, F-69270 Fontaines-Saint-Martin (FR)
(74) Mandataire: Dutruc-Rosset, Marie-Claude

(56) Documents cités:
- EP-A- 0 061 004
- EP-A- 0 155 335
- EP-A- 0 277 894
- EP-A- 0 353 755
- CH-A- 620 193
- PATENT ABSTRACTS OF JAPAN vol. 3, no. 27 (C-39)(99) 7 Mars 1979
- Houben Weyl, "Methoden der Organischen Chemie" Band E4 p. 64,65

## Description

La présente invention a pour objet un procédé de préparation de composés aromatiques polyalkoxylés, à partir de composés aromatiques porteurs d'au moins un atome d'halogène et d'au moins un groupe hydroxyle et, plus particulièrement, de dialkoxy-3,4 benzaldéhydes et de trialkoxy-3,4,5 benzaldéhydes.

L'invention vise plus particulièrement la préparation de triméthoxy-3,4,5 benzaldéhyde à partir de bromo-5 hydroxy-4 méthoxy-3 benzaldéhyde dénommé couramment "bromo-5 vanilline" et de diméthoxy-3,4 benzaldéhyde appelé "vératraldéhyde".

Dans l'exposé qui suit de la présente invention, on entend par "composé aromatique", la notion classique d'aromaticité telle que définie dans la littérature, notamment par Jerry MARCH - Advanced Organic Chemistry, 3ème édition, John Wiley and Sons, 1985 P. 37 et suivantes.

On définit par "composé aromatique polyalkoxylé", un composé aromatique porteur d'au moins deux radicaux alkoxy.

Dans le présent texte, on désigne d'une manière simplifiée par radicaux alkoxy, des radicaux du type R-O- dans lesquels R représente aussi bien un radical aliphatique, saturé, ou insaturé qu'un radical cycloaliphatique, saturé ou insaturé ou qu'un radical aliphatique porteur d'un carbocycle saturé, insaturé ou aromatique.

Dans tous les cas, la substitution de l'atome d'halogène par le radical R-O- est dénommé ci-après "réaction d'alkoxylation", quelle que soit la nature du radical R.

Dans la demande de brevet FR 2 663 925, on a décrit un procédé de préparation de triméthoxy-3,4,5 benzaldéhyde, à partir de bromo-5 vanilline, qui consiste :
- à faire réagir dans une première étape, la bromo-5 vanilline avec un alcoolate de métal alcalin, de préférence le méthylate de sodium, en présence d'un catalyseur au cuivre et d'un co-catalyseur réalisant ainsi une réaction de méthoxylation de la bromo-5 vanilline,
- à conduire dans une deuxième étape, la O-alkylation de l'hydroxy-4 diméthoxy-3,5 benzaldéhyde (syringaldéhyde) précédemment obtenu par addition directement dans le milieu réactionnel, d'un agent d'alkylation, de préférence, le chlorure de méthyle, ce qui conduit à l'obtention du triméthoxy-3,4,5 benzaldéhyde.

Dans la première étape du procédé décrit dans FR 2 663 925, la quantité d'alcoolate de métal alcalin mise en oeuvre représente de 1 à 4 fois, de préférence de 1,5 à 3 fois, la quantité stoechiométrique nécessaire, d'une part pour salifier le groupe hydroxyle et d'autre part pour transformer l'atome d'halogène en groupements méthoxy. Il s'avère très souhaitable et parfois nécessaire pour avoir un taux de transformation complet de la bromo-5 vanilline, d'engager un excès de méthylate de sodium.

Un inconvénient majeur de la mise en oeuvre d'un excès de méthylate de sodium est qu'il entraîne un coût supplémentaire de réactif et, de plus, nécessite des traitements ultérieurs de neutralisation pour l'éliminer sous forme, d'une part, de méthanol et, d'autre part, d'un hydroxyde ou d'un sel de sodium, par exemple, le sulfate de sodium, lors du traitement du milieu réactionnel par l'acide sulfurique.

De plus, l'excès de méthylate de sodium consomme une partie de l'agent d'alkylation, le chlorure de méthyle conduisant ainsi à la formation dans le milieu réactionnel, de diméthyléther qui, lorsqu'il est présent en quantités importantes, complique alors la mise en oeuvre du procédé en raison de risques d'explosion et de la nécessité de séparer le diméthyléther formé dans le milieu réactionnel.

On a proposé selon EP-A 0 155 335 d'éliminer l'excès d'alcoolate de métal alcalin par mise en oeuvre d'un chlorure d'acide tel que l'oxychlorure de phosphore.

Par ailleurs, il est connu selon l'ouvrage Methoden der Organischen Chemie (Houben-Weyl) Band E4, pp. 64-65 (1983) de faire réagir le dioxyde de carbone avec un alcoolate de métal alcalin afin d'obtenir le sel alcalin de carbonate d'alkyle.

Un des objectifs de la présente invention est de fournir un procédé perfectionné permettant de pallier aux inconvénients précités.

Il a maintenant été trouvé et c'est ce qui constitue l'objet de la présente invention, un procédé de préparation d'un composé aromatique polyalkoxylé, à partir d'un composé aromatique porteur d'au moins un atome d'halogène et d'au moins un groupe hydroxyle qui consiste à effectuer :
- une réaction d'alkoxylation en faisant réagir ledit composé avec un excès d'alcoolate de métal alcalin ou alcalino-terreux, en présence du cuivre et/ou d'un composé du cuivre, éventuellement en présence d'un co-catalyseur,
- une réaction de O-alkylation en faisant réagir le composé aromatique ainsi obtenu, porteur d'au moins un groupe alkoxy et d'au moins un groupe hydroxyle, avec un agent d'alkylation choisi parmi les halogénures d'alkyle inférieurs, les dialkylsulfates, les dialkylcarbonates, et qui est caractérisé par le fait que l'on introduit, en fin de réaction d'alkoxylation, du dioxyde de carbone en une quantité correspondant à la quantité d'alcoolate de métal alcalin ou alcalino-terreux qui n'a pas réagi.

Conformément au procédé de la présente invention, on transforme l'excès d'alcoolate de métal alcalin ou alcalino-terreux en agent d'alkylation, en le faisant réagir d'abord avec le dioxyde de carbone conduisant ainsi à un carbonate organique de métal alcalin ou alcalino-terreux qui réagit ensuite avec l'agent d'alkylation générant ainsi un agent d'alkylation qui peut être de nature différente.

A titre d'exemples, et sans limiter la portée de l'invention à cette interprétation, on donne un schéma réactionnel dans le cas de l'alkoxylation par le méthylate de sodium et l'alkylation par le chlorure de méthyle.

Ainsi, l'excès de méthylate de sodium est transformé en agent d'alkylation selon les deux réactions :

CH₃-O⁻Na⁺ + CO₂ ―> CH₃-O-CO-O⁻Na⁺

CH₃-O-CO-O⁻Na⁺ + CH₃-Cl ―> CH₃-O-CO-O-CH₃ + Na⁺Cl⁻

Le procédé de l'invention permet ainsi d'éliminer de manière astucieuse l'excès d'alcoolate de sodium mis en oeuvre en le transformant en diméthylcarbonate qui sert ainsi d'agent d'alkylation dans la dernière étape du procédé de l'invention.

La présente invention s'inscrit donc dans un procédé comportant une première étape d'alkoxylation d'un composé aromatique porteur d'au moins un atome d'halogène et d'au moins un groupe hydroxyle à l'aide d'un alcoolate de métal alcalin ou alcalino-terreux, puis une deuxième étape de O-alkylation du groupe hydroxyle et permet ainsi d'éliminer les inconvénients liés à la présence d'un excès d'alcoolate dans la première étape

Plus précisément, l'invention consiste en un procédé de préparation de composés aromatiques polyalkoxylés de formule générale (I) : 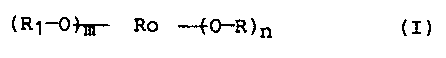
dans laquelle:
- m et n sont des nombres entiers supérieurs ou égaux à 1 avec, de préférence, m + n compris entre 2 et 6,
- Rₒ représente un radical cyclique aromatique ayant au moins 5 atomes dans le cycle, éventuellement substitué, et représentant au moins l'un des radicaux suivants :
   . un radical carbocyclique aromatique, monocyclique ou polycyclique,
   . un radical hétérocyclique aromatique, monocyclique ou polycyclique comportant au moins un des hétéroatomes O, N et S,
- R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique- ou arylaliphatique, saturé ou insaturé, linéaire ou ramifié,
- R₁ est un radical alkyle, linéaire ou ramifié, ayant de 1 à 6 atomes de carbone.

Conformément au procédé de l'invention, afin d'obtenir un composé répondant à la formule (I), on effectue dans une première étape, la réaction d'alkoxylation d'un composé aromatique porteur au moins d'un atome d'halogène et d'au moins un groupe hydroxyle.

A cet effet, on fait réagir en présence de cuivre et/ou d'un composé du cuivre :
- un composé aromatique porteur d'au moins un atome d'halogène et d'au moins un groupe hydroxyle répondant à la formule générale (II) : 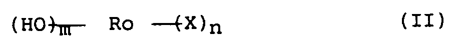 dans laquelle :
- m et n sont des nombres entiers supérieurs ou égaux à 1 avec, de préférence, m + n compris entre 2 et 6,
- X représente un atome d'iode, de brome ou de chlore,
- Rₒ représente un radical cyclique aromatique ayant au moins 5 atomes dans le cycle, éventuellement substitué, et représentant au moins l'un des radicaux suivants :
   . un radical carbocyclique aromatique, monocyclique ou polycyclique,
   . un radical hétérocyclique aromatique, monocyclique ou polycyclique comportant au moins un des hétéroatomes O, N et S,
- et un alcoolate de métal alcalin ou alcalino-terreux de formule générale (III) :

   M^{w+} [ O - R ]_{w}⁻ (III)

   dans laquelle :
- M représente un métal alcalin ou alcalino-terreux,
- w représente la valence du métal alcalin ou alcalino-terreux,
- R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique- ou arylaliphatique, saturé ou insaturé, linéaire ou ramifié.

Le terme "alcoolate" est utilisé dans le présent texte de manière générique et désigne également les aralkoxydes métalliques.

Dans l'étape suivante, on introduit du dioxyde de carbone afin de transformer l'excès l'alcoolate de métal alcalin ou alcalino-terreux en un carbonate organique de métal alcalin ou alcalino-terreux.

Ensuite, on introduit un agent d'alkylation tel que précédemment défini et on effectue ainsi la O-alkylation du produit précédemment obtenu.

L'invention s'applique plus particulièrement aux composés aromatiques porteurs d'au moins un atome d'halogène et d'au moins un groupe hydroxyle de formule (II) dans laquelle le radical Rₒ symbolise :
1° - un radical carbocyclique aromatique, monocyclique ou polycyclique. Par "radical carbocyclique polycyclique", on entend :
   . un radical constitué par au moins 2 carbocycles aromatiques et formant entre eux des systèmes ortho ou ortho et péricondensés,
   . un radical constitué par au moins 2 carbocycles dont l'un seul d'entre eux est aromatique et formant entre eux des systèmes ortho ou ortho et péricondensés.
2° - un radical hétérocyclique aromatique, monocyclique ou polycyclique. Par "radical hétérocyclique polycyclique", on définit :
   . un radical constitué par au moins 2 hétérocycliques contenant au moins un hétéroatome dans chaque cycle dont au moins l'un des deux cycles est aromatique et formant entre eux des systèmes ortho ou ortho et péricondensés,
   . un radical constitué par au moins un cycle hydrocarboné et au moins un hétérocycle dont au moins l'un des cycles est aromatique et formant entre eux des systèmes ortho ou ortho et péricondensés.
3° - un radical divalent constitué par un enchaînement de groupements, tels que définis aux paragraphes 1 et/ou 2 liés entre eux :
   . par un lien valentiel,
   . par un radical alkylène ou alkylidène ayant de 1 à 4 atomes de carbone, de préférence un radical méthylène ou isopropylidène,
   . par l'un des groupes suivants :
      -O- , -CO- ,
      -S- , -SO- , -SO₂- , 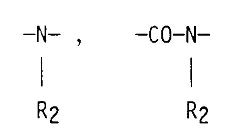 dans ces formules, R₂ représentant un atome d'hydrogène ou un radical alkyle ayant de 1 à 4 atomes de carbone, un radical cyclohexyle ou phényle.

Comme exemples de radicaux listés sous 1° à 3°, on peut citer :
1° - les radicaux phénylène, tolylène, xylylène, naphtylène,
2° - les radicaux furannediyle, pyrrolediyle, thiofènediyle, isoxazolediyle, furazannediyle, isothiazolediyle, imidazolediyle, pyrazolediyle, pyridinediyle, pyridazinediyle, pyrimidinediyle ; les radicaux naphtyridine-1,8 diyle, quinoléïnediyle, indolediyle, benzofurannediyle.
3° - les radicaux biphénylène, méthylène-1,1' biphénylène, isopropylidène-1,1' biphénylène, oxy-1,1' biphénylène, imino-1,1' biphénylène.

Les composés préférés sont ceux répondant à la formule (II) dans laquelle Ro représente un noyau benzénique.

Comme mentionné précédemment, le radical Rₒ qui est un radical aromatique porteur d'au moins un groupe hydroxyle et d'au moins un atome d'halogène, peut être également porteur d'un ou plusieurs autres substituants qui peuvent être un autre groupe hydroxyle ou un autre atome d'halogène ou de toute autre nature dans la mesure où ils ne gênent pas la réaction. Généralement, par plusieurs substituants, on définit moins de quatre substituants sur un noyau aromatique.

Comme exemples de substituants donnés à titre illustratif et sans caractère limitatif, on peut mentionner l'un des groupes ou fonctions suivantes :
. un radical de formule -R₃-OH dans laquelle le radical R₃ représente un lien valentiel ou un radical hydrocarboné divalent, linéaire ou ramifié, saturé ou insaturé ayant de 1 à 4 atomes de carbone, tel que par exemple, méthylène, éthylène, propylène, isopropylène, isopropylidène,
. un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, secbutyle, tert-butyle,
. un radical alcényle linéaire ou ramifié ayant de 2 à 6 atomes de carbone, de préférence, de 2 à 4 atomes de carbone, tel que vinyle, allyle,
. un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
. un groupe -CHO,
. un groupe acyle ayant de 2 à 6 atomes de carbone,
. un radical de formule -R₃-COOH, R₃ ayant la signification donnée précédemment,
. un radical de formule -R₃-COOR₄ dans laquelle R₃ a la signification donnée précédemment et R₄ représente un radical alkyle, linéaire ou ramifié, ayant de 1 à 6 atomes de carbone,
. un radical de formule -R₃-NH₂ avec un groupe NH₂ N-protégé, R₃ ayant la signification donnée précédemment,
. un radical de formule -R₃-N(R′₄)₂ dans laquelle R₃ a la signification donnée précédemment et les radicaux R′₄ identiques ou différents représentent un atome d'hydrogène ou un radical alkyle, linéaire ou ramifié, ayant de 1 à 6 atomes de carbone,
. un radical de formule R₃-CO-N(R′₄)₂, R₃ et R′₄ ayant la signification donnée précédemment,
. un radical de formule -R₃-Z dans laquelle R₃ a la signification donnée précédemment et Z symbolise un atome d'halogène X, tel que précédemment défini, un atome de fluor ou un groupe CF₃.

S'il y a présence de substituants sur le cycle aromatique, il y a lieu de veiller que celui-ci n'interfère pas au niveau du produit désiré.

De même, lors de la présence d'une fonction amine primaire, il peut être nécessaire de la N-protéger par un groupe protecteur, par exemple acyle, et plus spécialement acétyle.

Si le cycle est porteur d'un groupe du type -R₃-COOR₄, le groupe R₄ sera échangé par le groupe R provenant de l'alcoolate de métal alcalin ou alcalino-terreux, si R₄ et R sont des groupes alkyles de nature différente.

Si le cycle est porteur d'une fonction acide, notamment d'un groupe du type -R₃-COOH, le groupe sera salifié et entraînera donc une consommation supplémentaire de l'alcoolate utilisé.

Lorsque le cycle aromatique est porteur d'une chaîne aliphatique latérale avec présence d'un atome d'halogène X, celui-ci peut être substitué également par le groupe R-0- provenant de l'alcoolate de métal alcalin ou alcalino-terreux et il y aura donc lieu d'en tenir compte pour la détermination des quantités de réactifs à mettre en oeuvre.

Comme exemples de substituants cités à titre préférentiel et sans caractère limitatif, on peut mentionner :
- un ou plusieurs radicaux alkyle ayant de 1 à 4 atomes de carbone,
- un ou plusieurs radicaux alkoxy ayant de 1 à 4 atomes de carbone,
- un ou plusieurs radicaux hydroxyle.

A titre d'exemples de composés aromatiques porteurs d'au moins un atome d'halogène et d'au moins un groupe hydroxyle répondant à la formule générale (II), on peut citer plus particulièrement :
- le bromo-2 phénol
- le bromo-3 phénol
- le bromo-4 phénol
- le bromo-1 naphtol-2
- le bromo-6 naphtol-2
- le bromo-2 méthyl-4 phénol
- le bromo-4 diméthyl-2,6 phénol
- le bromo-4 diméthyl-3,5 phénol
- le dibromo-2,6 méthyl-4 phénol
- le bromo-2-p-crésol
- le bromo-2 chloro-4 phénol
- le bromo-4 chloro-2 phénol
- le bromo-4 chloro-6-o-crésol
- les bromofluorophénols
- les bromo bis-phénols
- les bromo isopropylidène-4,4′ bis-phénols

L'alcoolate de métal alcalin ou alcalino-terreux qui intervient dans le procédé de l'invention répond à la formule (III) dans laquelle R représente :
1° - un radical alkyle, alcényle, alcadiényle, alcynyle linéaire ou ramifié ayant, de préférence, moins de 6 atomes de carbone,
2° - un radical cycloalkyle ou cycloalcényle ayant, de préférence, de 5 à 7 atomes de carbone et l'on peut citer en particulier le radical cyclohexyle,
3° - un radical aliphatique acyclique, saturé ou insaturé, linéaire ou ramifié tel que précisé sous 1°, porteur d'un substituant cyclique. Par cycle, on entend un cycle carbocyclique saturé, insaturé ou aromatique. Comme exemple, on peut mentionner le radical benzyle.

Parmi les alcoolates précités, on met en oeuvre de préférence dans le procédé de l'invention, les alcoolates de métaux alcalins et plus particulièrement les alcoolates de sodium ou de potassium des alcanols primaires ou secondaires ayant 1 à 4 atomes de carbone conviennent particulièrement bien.

Les plus fréquemment utilisés sont le méthylate de sodium et l'éthylate de sodium.

Un mode de mise en oeuvre préférentiel de l'invention réside dans un procédé de préparation d'un composé aromatique polyalkoxylé répondant à la formule générale (Ia) : 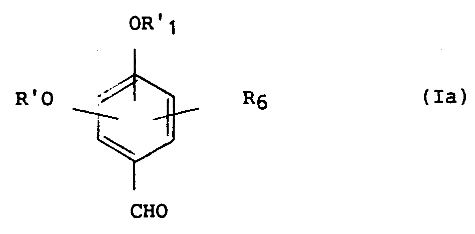
dans laquelle:
- R′, R′₁, R₆ identiques ou différents représentent un radical alkyle, linéaire ou ramifié, ayant de 1 à 4 atomes de carbone,
- R₆ pouvant représenter également un atome d'hydrogène, un radical alkoxy, linéaire ou ramifié ayant de 1 à 4 atomes de carbone.

Le procédé de préparation d'un composé aromatique polyalkoxylé répondant à la formule générale (Ia) comporte les étapes suivantes :
1° - On fait réagir un composé aromatique entrant dans la définition générale de la formule (II) qui est un halogéno hydroxy benzaldéhyde répondant à la formule générale (IIa) : 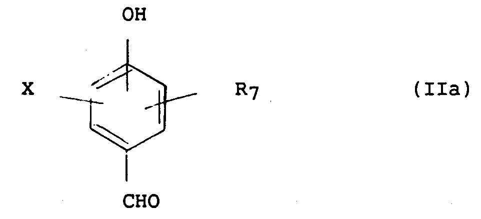 dans laquelle :
   - X représente un atome d'iode, de brome ou de chlore,
   - R₇ représente un atome d'hydrogène, un atome d'iode, de brome, un atome de chlore, un radical alkyle ayant de 1 à 4 atomes de carbone, un radical alkoxy ayant de 1 à 4 atomes de carbone, un radical hydroxyle,
   dans ladite formule (IIa), le radical hydroxyle pouvant être en position ortho-, méta- ou para- de la fonction aldéhyde,
   avec un alcoolate de métal alcalin ou alcalino-terreux ayant de 1 à 4 atomes de carbone, en présence d'un catalyseur au cuivre et, éventuellement d'un co-catalyseur,
2° - En fin de réaction d'alkoxylation, on introduit du dioxyde, de carbone en une quantité correspondant à la quantité l'alcoolate de métal alcalin ou alcalino-terreux qui n'a pas réagi,
3° - On réalise la O-alkylation du produit obtenu précédemment après alkoxylation, par addition dans le milieu réactionnel organique, d'un agent d'alkylation apportant le reste alkyle R′₁ ayant de 1 à 4 atomes de carbone.

Comme exemples de substrats mis en oeuvre préférentiellement, on peut citer plus particulièrement les composés répondant à la formule générale (IIa) dans laquelle :
. Groupe I :
   - le radical OH est en position para par rapport à la fonction CHO,
   - l'atome d'halogène X est en position ortho par rapport au radical OH,
   - le radical R₇ est en position ortho par rapport au radical OH.
. Groupe II :
   - le radical OH est en position ortho par rapport à la fonction CHO,
   - l'atome d'halogène X est en position para par rapport au radical OH,
   - le radical R₇ est en position ortho par rapport au radical OH.
. Groupe III :
   - le radical OH est en position ortho par rapport à la fonction CHO,
   - l'atome d'halogène X est en position ortho par rapport au radical OH,
   - le radical R₇ est en position para par rapport au radical OH,
. Groupe IV :
   - le radical OH est en position méta par rapport à la fonction CHO,
   - l'atome d'halogène X est en position ortho par rapport au radical OH,
   - le radical R₇ est en position para par rapport au radical OH.
. Groupe V :
   - le radical OH est en position méta par rapport à la fonction CHO,
   - l'atome d'halogène X est en position para par rapport au radical OH,
   - le radical R₇ est en position ortho par rapport au radical OH.

Les composés de formule (IIa) mis en oeuvre préférentiellement, répondent plus précisément aux formules suivantes: 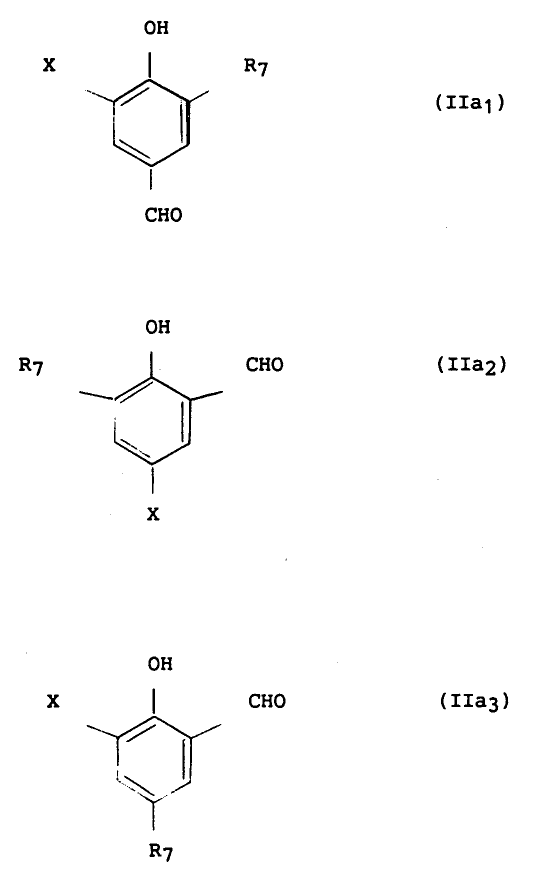
dans lesquelles :
- X et R₇ ont les significations données précédemment.

A titre d'exemples d'halogéno hydroxy benzaldéhydes répondant à la formule (IIa) qui servent de substrats de départ dans le présent procédé, on peut citer plus précisément :
- le bromo-3 hydroxy-4 benzaldéhyde,
- le iodo-3 hydroxy-4 benzaldéhyde
- le dibromo-3,5 hydroxy-4 benzaldéhyde
- le diiodo-3,5 hydroxy-4 benzaldéhyde
- le bromo-5 méthoxy-3 hydroxy-4 benzaldéhyde
- le iodo-5 méthoxy-3 hydroxy-4 benzaldéhyde
- le bromo-5 éthoxy-3 hydroxy-4 benzaldéhyde
- le iodo-5 éthoxy-3 hydroxy-4 benzaldéhyde
- le bromo-3 dihydroxy-4,5 benzaldéhyde
- le iodo-3 dihydroxy-4,5 benzaldéhyde
- le bromo-3 dihydroxy-2,5 benzaldéhyde
- le iodo-3 dihydroxy-2,5 benzaldéhyde
- le bromo-2 hydroxy-4 benzaldéhyde
- le iodo-2 hydroxy-4 benzaldéhyde
- le bromo-4 hydroxy-3 benzaldéhyde
- le iodo-4 hydroxy-3 benzaldéhyde
- le bromo-3 hydroxy-2 benzaldéhyde
- le iodo-3 hydroxy-2 benzaldéhyde
- le bromo-5 hydroxy-2 benzaldéhyde
- le iodo-5 hydroxy-2 benzaldéhyde
Dans ce mode préférentiel de mise en oeuvre du procédé de l'invention, l'alcoolate de métal alcalin ou alcalino-terreux de formule (III) mis en oeuvre est de préférence un alcoolate de sodium ou potassium d'un alcanol primaire ou secondaire ayant de 1 à 4 atomes de carbone et, de préférence, le méthylate ou l'éthylate de sodium.

Conformément au procédé de l'invention, on fait réagir le composé aromatique porteur d'au moins un atome d'halogène et d'au moins un groupe hydroxyle répondant à la formule (II) avec un alcoolate de métal alcalin ou alcalino-terreux de formule (III), en présence d'un catalyseur au cuivre.

Les composés du cuivre servant de catalyseurs sont connus. Ce sont d'une manière générale tous les composés organiques ou inorganiques du cuivre I ou du cuivre II.

Le cuivre métal peut être utilisé, mais son action est plus lente car il faut préalablement qu'il se transforme en partie en cuivre I ou cuivre II.

A titre non limitatif, on peut citer comme composés du cuivre, le chlorure cuivreux, le chlorure cuivrique, le carbonate de cuivre II basique, le nitrate cuivreux, le nitrate cuivrique, le sulfate cuivrique, l'acétate cuivrique, le trifluorométhylsulfonate cuivrique, l'hydroxyde cuivrique, le picolinate de cuivre II, le méthylate de cuivre I, le méthylate de cuivre II, le chélate de cuivre II de la 8-quinoléine, les composés de cuivre de formule ClCuOCH₃, Cu₂(OCH₃)₂(acac)₂.

Une variante préférée du procédé de l'invention consiste à faire appel également à un co-catalyseur.

A titre d'exemples de co-catalyseurs utilisés préférentiellement dans le procédé de l'invention, on peut citer un co-catalyseur choisi parmi les carbonates organiques, les carbonates mixtes organo-métalliques, le dioxyde de carbone ou les composés susceptibles de former du dioxyde de carbone.

Les carbonates organiques et les carbonates mixtes organo-métalliques utilisés dans l'invention sont plus particulièrement les carbonates de formule générale (IV) :

[R₈ - O - CO - O -]ₚ - R₉ (IV)

dans laquelle :
- R₈ représente :
   . un radical alkyle, linéaire ou ramifié, ayant 1 à 6 atomes de carbone,
   . un radical cycloalkyle ayant 5 à 6 atomes de carbone,
   . un radical cycloalkyle, ayant 5 ou 6 atomes de carbone substitué par un ou deux radicaux alkyle ayant 1 à 12 atomes de carbone,
   . un radical aryle ayant de 6 à 12 atomes de carbone,
   . un radical aryle substitué par 1 ou 2 radicaux alkyle ayant 1 à 12 atomes de carbone ;
   . un radical R₁₀ - O - CO - dans lequel R₁₀ représente un radical alkyle, linéaire ou ramifié ayant 1 à 6 atomes de carbone, un radical aryle ayant de 6 à 12 atomes de carbone, un radical cycloalkyle ayant 5 ou 6 atomes de carbone ;
- R₉ représente :
   . un radical alkyle, linéaire ou ramifié, ayant 1 à 6 atomes de carbone,
   . un radical cycloalkyle ayant 5 ou 6 atomes de carbone,
   . un radical cycloalkyle ayant 5 ou 6 atomes de carbone substitué par un ou deux radicaux alkyle ayant 1 à 12 atomes de carbone,
   . un radical aryle ayant 6 à 12 atomes de carbone,
   . un radical aryle substitué par 1 ou 2 radicaux alkyle ayant 1 à 12 atomes de carbone,
   . un métal alcalin ou alcalino-terreux, de préférence le sodium ou le potassium.
- p = 1 ou p = 2 lorsque R₉ représente un métal alcalino-terreux ;
- R₈ et R₉ peuvent former ensemble un radical alkylène ayant 2 à 6 atomes de carbone.

De préférence, les carbonates organiques et les carbonates mixtes organo-métalliques sont les carbonates de formule générale (IVa) :

R₈ - O - CO - O - R₉ (IVa)

dans laquelle :
- R₈ représente :
   . un radical alkyle, linéaire ou ramifié, ayant 1 à 6 atomes de carbone,
   . un radical cycloalkyle ayant 5 à 6 atomes de carbone,
   . un radical cycloalkyle, ayant 5 ou 6 atomes de carbone substitué par un ou deux radicaux alkyle ayant 1 à 4 atomes de carbone,
   . un radical phényle,
   . un radical phényle substitué par 1 ou 2 radicaux alkyle ayant 1 à 4 atomes de carbone,
   . un radical R₁₀ - O - CO - dans lequel R₁₀ représente un radical alkyle, linéaire ou ramifié ayant 1 à 6 atomes de carbone, un radical phényle, un radical cycloalkyle ayant 5 ou 6 atomes de carbone
- R₉ représente :
   . un radical alkyle, linéaire ou ramifié, ayant 1 à 6 atomes de carbone,
   . un radical cycloalkyle ayant 5 ou 6 atomes de carbone,
   . un radical cycloalkyle ayant 5 ou 6 atomes de carbone substitué par un ou deux radicaux alkyle ayant 1 à 4 atomes de carbone,
   . un radical phényle,
   . un radical phényle substitué par un ou deux radicaux alkyle ayant 1 à 4 atomes de carbone,
   . un atome de sodium ou de potassium,
- R₈ et R₉ peuvent former ensemble un radical alkylène ayant 2 à 6 atomes de carbone.

Comme exemples de carbonates organiques ou organo-métalliques, on peut citer : le carbonate de ditertiobutyle, le carbonate de diéthyle, le carbonate de diméthyle, le carbonate d'éthylène, le carbonate de propylène, le carbonate de phényle et de tertiobutyle, le carbonate de tertiobutyle et de sodium, le dicarbonate de ditertiobutyle.

En ce qui concerne le dioxyde de carbone, il peut être mis en oeuvre en solution dans le milieu réactionnel, par circulation dans celui-ci.

Comme exemples de composés susceptibles de former du dioxyde de carbone dans le milieu réactionnel, on peut citer :
- les α(ou β)cétoesters et les α(ou β)cétoacides ;
- les carbonates d'amine, les urées et les carbodiimides ;
- les acides dicarboxyliques comme l'acide malonique, les acides α-cyano- ou α-nitro-carboxyliques ;
- les acides carboxyliques βγ-insaturés.

Conformément au procédé de l'invention, l'alkoxylation du composé aromatique porteur d'au moins un atome d'halogène et d'au moins un groupe hydroxyle répondant à la formule (II) est conduite en milieu organique constitué, le plus souvent, par l'alcanol correspondant à l'alcoolate de métal alcalin ou alcalino-terreux utilisé.

On choisit, préférentiellement comme solvant, le méthanol ou l'éthanol.

Lorsque l'alcoolate de métal alcalin ou alcalino-terreux présente une condensation en carbone supérieure à 4 atomes de carbone, il est souhaitable de faire appel à un solvant inerte vis-à-vis de la réaction et de choisir, de préférence, un solvant aprotique polaire.

Comme exemples de solvants aprotiques polaires convenant à la mise en oeuvre du procédé de l'invention, on peut mentionner, plus particulièrement les éthers et, plus précisément, les éthers diméthyliques dérivant de l'oxyde d'éthylène ou de l'oxyde de propylène tels que le diméthyléther de l'éthylèneglycol (ou diméthoxy-1,2 éthane), le diméthyléther du diéthylèneglycol (ou diméthoxy-1,5 oxa-3 pentane), le diméthoxy-1,8 dioxa-3,6 octane, le diméthoxy-1,11 trioxa-3,6,9 undécane, le diméthoxy-1,2 méthyl-1 éthane, le diméthoxy-1,5 diméthyl-1,4 oxa-3 pentane, le diméthoxy-1,7 diméthyl-1,4 dioxa-3,6 octane.

On peut également utiliser plusieurs solvants.

Parmi tous les solvants précités, le diméthyléther de l'éthylène-glycol et le diméthyléther du diéthylèneglycol sont préférés.

La concentration du composé de formule (II) exprimée en poids dudit composé (II) par rapport au poids total composé (II) + solvant est généralement de 3 à 40 % et, de préférence, de 10 à 30 %.

La quantité d'alcoolate de métal alcalin ou alcalino-terreux utilisée est supérieure à la quantité stoechiométrique nécessaire, d'une part pour transformer le ou les atomes d'halogène en groupements alkoxy et d'autre part pour transformer le composé de formule (II) en phénate de métal alcalin ou alcalino-terreux dans le cas où ledit composé présente un ou plusieurs radicaux hydroxyle. On effectue donc la salification du ou des groupements OH.

Généralement, l'alcoolate de métal alcalin ou alcalino-terreux est mis en oeuvre en une quantité correspondant à la quantité stoechiométrique nécessaire pour salifier le ou les groupes hydroxyle plus la quantité égale de 1,1 à 5 fois, et de préférence de 1,5 à 3 fois, la quantité stoechiométrique nécessaire pour transformer le ou les atomes d'halogène en groupe alkoxy.

La concentration de l'alcoolate de métal alcalin ou alcalino-terreux est avantageusement supérieure à 1 mole/litre et, de préférence comprise entre 1 et 5 moles/litre.
Il est à noter que la borne supérieure ne présente aucun caractère critique.

De manière pratique, l'alcoolate de métal alcalin ou alcalino-terreux est formé in situ, en faisant réagir un excès d'alcanol avec le métal alcalin ou alcalino-terreux choisi.

La quantité de catalyseur au cuivre utilisée dans le procédé de l'invention peut varier très largement.

Habituellement, le rapport molaire catalyseur au cuivre/composé de formule (II) est de 1 à 50 % et, de préférence, de 2 % à 20 %.

Selon une variante préférée du procédé de l'invention, on met en oeuvre un co-catalyseur en une quantité telle que l'on ait un rapport molaire co-catalyseur/catalyseur au cuivre de 1 à 10 et, de préférence, de 1 à 5.

La température de la réaction d'alkoxylation est généralement comprise entre 60°C et 220°C et, de préférence, entre 100°C et 180°C.

La pression n'est pas un paramètre critique en soi, mais pour atteindre les températures indiquées précédemment et ne pas perdre de solvant, le procédé est habituellement réalisé sous pression autogène.

Généralement, cette pression autogène du mélange réactionnel est inférieure ou égale à 5 MPa (50 bars).

La durée de la réaction d'alkoxylation peut varier largement entre 1 et 10 heures, de préférence entre 2 et 5 heures.

En fin de ladite réaction, la caractéristique du procédé de l'invention est d'introduire du gaz carbonique dans le milieu réactionnel afin d'effectuer la carbonatation de l'alcoolate de métal alcalin ou alcalino-terreux.

Un mode d'exécution de l'invention consiste à introduire le dioxyde de carbone, dans le milieu réactionnel sous forme gazeuse.

L'introduction du dioxyde de carbone peut se faire dans le milieu réactionnel maintenu à la température d'alkoxylation telle que précédemment définie ou à une température qui peut être légèrement inférieure de 10°C à 30°C ce qui implique, dans ce cas, un refroidissement.

La quantité de gaz carbonique mise en oeuvre est, de préférence, au moins égale à la quantité stoechiométrique d'alcoolate de métal alcalin ou alcalino-terreux qui n'a pas réagi.

On introduit généralement une quantité de dioxyde de carbone exprimée par rapport à l'alcoolate allant de la quantité stoechiométrique à un excès pouvant atteindre 50 % mais, de préférence, compris entre 10 et 20 %.

Après introduction du gaz carbonique, on maintient le milieu réactionnel sous agitation et à une température comprise dans l'intervalle précité, pendant une courte durée, non critique, d'environ 5 à 30 minutes.

Dans l'étape suivante du procédé de l'invention, on conduit la réaction de O-alkylation du composé aromatique précédemment obtenu qui est porteur d'au moins un groupe alkoxy et d'au moins un groupe hydroxyle.

A cet effet, on le met en contact avec un agent d'alkylation. On peut faire appel aux alkylsulfates de formule R₁-O-SO₂-O-R₁ ou aux alkylcarbonates de formule R₁-0-CO-O-R₁, dans lesdites formules, R₁ représente un radical alkyle, linéaire ou ramifié, ayant de 1 à 6 atomes de carbone.

Parmi les agents d'alkylation précités, le diméthylsulfate et le diméthylcarbonate sont préférés.

Toutefois, on préfère selon l'invention choisir, à titre d'agent d'alkylation, un halogénure d'alkyle inférieur répondant à la formule générale (V) :

R₁-X (V)

dans ladite formule, X représente un atome de brome, de chlore ou d'iode et R₁ représente un radical alkyle linéaire ou ramifié ayant de 1 à 6 atomes de carbone et, de préférence, de 1 à 4 atomes de carbone.

Parmi les halogénures de formule (V), on préfère mettre en oeuvre ceux répondant à la formule (V) dans laquelle X est un atome de chlore et R′₁ un radical alkyle, linéaire ou ramifié, ayant de 1 à 4 atomes de carbone.

Le plus souvent, on utilise un halogénure de méthyle ou un halogénure d'éthyle.

Parmi les halogénures, les chlorures, bromures et iodures sont généralement mis en oeuvre et plus spécifiquement encore le chlorure de méthyle, le chloroéthane, le bromure de méthyle et le bromoéthane.

En raison de leur plus faible coût, on préfère utiliser le chlorure de méthyle et le chloroéthane.

Une variante d'exécution de l'invention consiste à ajouter dans le milieu réactionnel, de préférence avant l'addition de l'agent d'alkylation, un sel sous forme d'iodure, afin d'avoir une cinétique plus élevée. Comme exemples de sels, on peut citer les iodures de métal alcalin, notamment de sodium, de potassium ou de lithium.

Pour ce qui est de la quantité des réactifs à mettre en oeuvre dans la dernière étape du procédé de l'invention, on définit ci-après les quantités préférées.

La quantité d'agent d'alkylation engagée est fonction du nombre de groupes hydroxyle présents sur le noyau aromatique du produit obtenu précédemment, après alkoxylation. Elle est de préférence au moins égale à quantité stoechiométrique jusqu'à un excès pouvant atteindre 100 %.

Elle est choisie, de préférence, égale à la quantité stoechiométrique.

A titre d'exemples, on précisera que le rapport molaire halogénure d'alkyle/alkoxy hydroxy benzaldéhyde est compris entre 1,0 et 2,0 et, de préférence, voisin de 1,0 et que le rapport molaire halogénure d'alkyle/alkoxy dihydroxy benzaldéhyde est compris entre 2,0 et 4,0 et, de préférence, voisin de 2,0.

La quantité d'agent d'alkylation mise en oeuvre est déterminée en tenant compte de la quantité excédentaire de l'alcoolate de métal alcalin ou alcalino-terreux.

Si la quantité excédentaire d'alcoolate de métal alcalin ou alcalino-terreux est supérieure à la quantité stoechiométrique d'agent d'alkylation requise pour la ou les réactions de O-alkylation, la quantité d'agent d'alkylation engagée est au moins égale à la quantité d'alcoolate de métal alcalin ou alcalino-terreux ou en excès pouvant atteindre 100 %.

Si la quantité excédentaire d'alcoolate de métal alcalin ou alcalino-terreux est inférieure à la quantité stoechiométrique d'agent d'alkylation requise pour la ou les réactions de O-alkylation, la quantité d'agent d'alkylation est égale ou en excès, par rapport à la stoechiométrie de la réaction de O-alkylation. On se réfèrera aux quantités telles que précédemment définies.

La quantité de sel sous forme d'iodure mise en oeuvre dans la variante préférée de l'invention peut varier dans de larges limites. Généralement, le rapport molaire entre le sel sous forme d'iodure et l'agent d'alkylation varie entre 0,05 et 0,20 et se situe, de préférence, aux environs de 0,10.

En ce qui concerne les conditions réactionnelles, la température de la réaction de 0-alkylation n'est pas critique ; elle a une influence sur la cinétique de la réaction. Généralement, on réalise la 0-alkylation entre 80°C et 200°C. De préférence, on opère à une température entre 100°C et 160°C.

La pression n'est pas critique. Elle a une incidence sur la cinétique de la réaction. Elle varie habituellement entre la pression atmosphérique et 50 bars et se situe, de préférence, entre la pression atmosphérique et 20 bars.

La pression est habituellement créée par le solvant réactionnel et l'agent d'alkylation servant à la 0-alkylation lorsqu'ils sont gazeux dans les conditions réactionnelles.

La durée de la réaction de O-alkylation est fonction notamment de la température réactionnelle. Elle varie le plus souvent entre 1 heure et 8 heures. Généralement, une durée de 1 à 4 heures est suffisante.

Selon un mode de réalisation pratique de l'invention, on peut mélanger tous les réactifs de la première étape à savoir le composé aromatique de formule (II), le catalyseur au cuivre et, éventuellement le co-catalyseur et après chauffage pendant la durée nécessaire à la réaction d'alkoxylation, ajouter dans le milieu réactionnel, le dioxyde de carbone, éventuellement un sel sous forme d'iodure, puis l'agent d'alkylation.

Un mode préféré de mise en pratique de l'invention consiste d'abord à préparer une solution de l'alcoolate de métal alcalin dans l'alcanol correspondant à raison de, par exemple, 20 à 50 % en poids.

Dans l'appareillage, on charge sous atmosphère de gaz inerte, de préférence, l'azote, le composé aromatique de formule (II), le catalyseur au cuivre puis, éventuellement le co-catalyseur qui est, de préférence, le dioxyde de carbone ou un carbonate organique, puis l'on introduit la solution de l'alcoolate de métal alcalin. Dans le cas du dioxyde de carbone, on envoie donc un courant gazeux dans le milieu réactionnel.

On porte ensuite le milieu réactionnel à la température choisie entre 60°C et 220°C, de préférence, entre 100°C et 180°C, pendant la durée pré-définie.

En fin de réaction, on introduit un courant de dioxyde de carbone en une quantité correspondant à la quantité d'alcoolate de métal alcalin résiduelle.

On introduit ensuite l'agent d'alkylation, de préférence l'halogénure d'alkyle qui peut être liquide ou gazeux.

On maintient le chauffage entre 80°C et 200°C, de préférence entre 100°C et 160°C, pendant la durée nécessaire à la réaction de 0-alkylation.

En fin de réaction, on sépare le composé aromatique polyalkoxylé de formule (I) obtenu, selon les techniques classiques de séparation, soit par distillation, soit par extraction par un solvant approprié, par exemple, le toluène dans le cas du triméthoxy-3,4,5 benzaldéhyde.

Un des avantages du procédé de l'invention est qu'il permet d'éviter les inconvénients liés à la présence d'un excès d'alcoolate de métal alcalin ou alcalino-terreux.

Le procédé de l'invention est particulièrement bien adapté à la préparation des alkoxybenzaldéhydes suivants :
- le triméthoxy-3,4,5 benzaldéhyde
- le diméthoxy-3,4 benzaldéhyde.
- le diéthoxy-3,4 benzaldéhyde
- l'éthoxy-3 méthoxy-4 benzaldéhyde

Le triméthoxy-3,4,5 benzaldéhyde qui peut être préparé selon le procédé de l'invention sert notamment pour la préparation de produits pharmaceutiques.

Les exemples qui suivent, illustrent l'invention sous toutefois la limiter.

Dans les exemples, les abréviations suivantes signifient
BHMB : bromo-5 hydroxy-4 méthoxy-3 benzaldéhyde
TMBA : triméthoxy-3,4,5 benzaldéhyde
syringaldéhyde : hydroxy-4 diméthoxy-3,5 benzaldéhyde

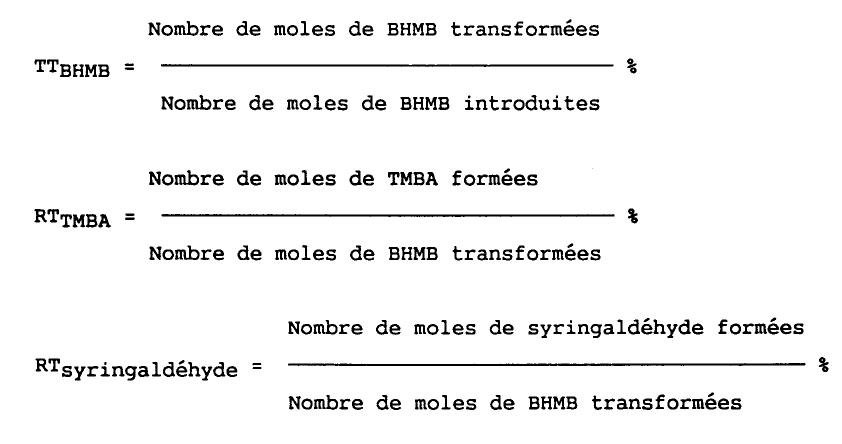

### EXEMPLE 1

Dans un autoclave muni d'un système de chauffage et d'une agitation, on charge sous atmosphère d'azote :
- 173,2 g (0,750 mole) de bromo-5 hydroxy-4 méthoxy-3 benzaldéhyde (BHMB)
- 162 g de méthylate de sodium
- 1000 cm³ (791 g) de méthanol
- 8,3 g de carbonate de cuivre II basique de formule CuCO₃,Cu(OH)₂
- 16 g de dioxyde de carbone

Le dioxyde de carbone est apporté sous forme gazeuse, par bullage dans le mélange réactionnel.

On chauffe sous agitation, pendant 3 heures, à 135°C, sous pression autogène, afin de réaliser la réaction de méthoxylation.

On refroidit le mélange réactionnel à 120°C.

On charge 60 g de dioxyde de carbone et l'on agite pendant 15 minutes.

On charge ensuite 80 g de chlorure de méthyle.

On maintient le mélange réactionnel à 160°C pendant 4 heures.

On refroidit à température ambiante.

On sépare par filtration la partie insoluble.

On dose par chromatographie liquide haute performance, le BHMB n'ayant pas réagi et le triméthoxy-3,4,5 benzaldéhyde obtenu.

Les résultats obtenus sont les suivants :
- taux de transformation du BHMB (TT_{BHMB} %) = 100 %
- rendement en TMBA (RT_{TMBA} ) = 81 %
- rendement en syringaldéhyde (RT_{syringaldéhyde} %) = 18 %

### EXEMPLE 2

On répète l'exemple 1 avec les mêmes charges, les mêmes conditions opératoires, mais en ajoutant 22,5 g d'iodure de sodium, juste avant l'introduction du chlorure de méthyle.

Les résultats obtenus sont les suivants :
- taux de transformation du BHMB (TT_{BHMB} %) = 100 %
- rendement en TMBA (RT_{TMBA}) = 85,4 %
- rendement en syringaldéhyde (RT_{syringaldéhyde} %) = 13 %

### EXEMPLE 3

On répète l'exemple 1 avec les mêmes charges, mais en modifiant les conditions opératoires.

On réalise la réaction de méthoxylation, en chauffant sous agitation pendant 3 heures, à 135°C, sous pression autogène.

On refroidit le mélange réactionnel à 120°C puis l'on charge 60 g de dioxyde de carbone et l'on agite pendant 15 minutes.

On charge ensuite 135 g de diméthylcarbonate.

On maintient le mélange réactionnel à 160°C pendant 6 heures.

On refroidit à température ambiante.

On sépare par filtration la partie insoluble.

On dose par chromatographie liquide haute performance, le BHMB n'ayant pas réagi et le TMBA obtenu.

Les résultats obtenus sont les suivants :
- taux de transformation du BHMB (TT_{BHMB} %) = 100 %
- rendement en TMBA (RT_{TMBA}) = 92 %
- rendement en syringaldéhyde (RT_{syringaldéhyde} %) = 7 %

## Revendications

1. Procédé de préparation d'un composé aromatique polyalkoxylé, à partir d'un composé aromatique porteur d'au moins un atome d'halogène et d'au moins un groupe hydroxyle qui consiste à effectuer :
- une réaction d'alkoxylation en faisant réagir ledit composé avec un excès d'alcoolate de métal alcalin ou alcalino-terreux, en présence du cuivre et/ou d'un composé du cuivre, éventuellement en présence d'un co-catalyseur,
- une réaction de O-alkylation en faisant réagir le composé aromatique ainsi obtenu, porteur d'au moins un groupe alkoxy et d'au moins un groupe hydroxyle, avec un agent d'alkylation choisi parmi les halogénures d'alkyle inférieurs, les dialkylsulfates, les dialkylcarbonates, et qui est caractérisé par le fait que l'on introduit, en fin de réaction d'alkoxylation, du dioxyde de carbone en une quantité correspondant à la quantité d'alcoolate de métal alcalin ou alcalino-terreux qui n'a pas réagi.

2. Procédé selon la revendication 1 caractérisé par le fait que l'on transforme l'excès d'alcoolate de métal alcalin ou alcalino-terreux en dialkylcarbonate utilisé comme agent d'alkylation, en le faisant réagir, en fin de réaction d'alkoxylation avec du dioxyde de carbone, puis avec un halogénure d'alkyle mis en oeuvre en quantité suffisante.

3. Procédé selon l'une des revendications 1 et 2 caractérisé par le fait que le composé aromatique polyalkoxylé répond à la formule générale (I) : 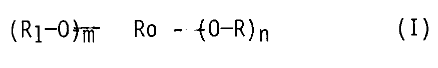 dans laquelle :
- m et n sont des nombres entiers supérieurs ou égaux à 1 avec, de préférence, m + n compris entre 2 et 6,
- Rₒ représente un radical cyclique aromatique ayant au moins 5 atomes dans le cycle, éventuellement substitué, et représentant au moins l'un des radicaux suivants :
. un radical carbocyclique aromatique, monocyclique ou polycyclique,
. un radical hétérocyclique aromatique, monocyclique ou polycyclique comportant au moins un des hétéroatomes O, N et S,
- R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique- ou arylaliphatique, saturé ou insaturé, linéaire ou ramifié,
- R₁ est un radical alkyle, linéaire ou ramifié, ayant de 1 à 6 atomes de carbone.

4. Procédé selon l'une des revendications 1 à 3 caractérisé par le fait que l'on effectue les étapes suivantes :
1° - On fait réagir un composé aromatique porteur d'au moins un atome d'halogène et d'au moins un groupe hydroxyle répondant à la formule générale (II) :
(HO)̵ₘ Ro (̵X)ₙ (II)
dans laquelle :
- m et n sont des nombres entiers supérieurs ou égaux à 1 avec, de préférence, m + n compris entre 2 et 6,
- X représente un atome d'iode, de brome ou de chlore,
- Rₒ représente un radical cyclique aromatique ayant au moins 5 atomes dans le cycle, éventuellement substitué, et représentant au moins l'un des radicaux suivants :
. un radical carbocyclique aromatique, monocyclique ou polycyclique,
. un radical hétérocyclique aromatique, monocyclique ou polycyclique comportant au moins un des hétéroatomes O, N et S,
- et un alcoolate de métal alcalin ou alcalino-terreux de formule générale (III) :
M^{w+} [ O - R ]_{w}⁻ (III)
dans laquelle :
- M représente un métal alcalin ou alcalino-terreux,
- w représente la valence du métal alcalin ou alcalino-terreux,
- R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique- ou arylaliphatique, saturé ou insaturé, linéaire ou ramifié.
en présence d'un catalyseur au cuivre et éventuellement d'un co-catalyseur
2° - En fin de réaction d'alkoxylation, on introduit du dioxyde de carbone,
3° - On réalise la O-alkylation du produit obtenu précédemment après alkoxylation, par addition dans le milieu réactionnel organique d'un agent d'alkylation choisi parmi les halogénures d'alkyle inférieurs, les dialkylsulfates, les dialkylcarbonates.

5. Procédé selon l'une des revendications 1 à 4 caractérisé par le fait que le composé aromatique porteur d'au moins un atome d'halogène et d'au moins un groupe hydroxyle répond à la formule générale (II) dans laquelle le radical Ro symbolise :
1° - un radical carbocyclique aromatique, monocyclique ou polycyclique,
2° - un radical hétérocyclique aromatique, monocyclique ou polycyclique,
3° - un radical divalent constitué par un enchaînement de groupements, tels que définis aux paragraphes 1 et/ou 2 liés entre eux :
. par un lien valentiel,
. par un radical alkylène ou alkylidène ayant de 1 à 4 atomes de carbone, de préférence un radical méthylène ou isopropylidène,
. par l'un des groupes suivants :
-O- , -CO- ,
-S- , -SO- , -SO₂- , 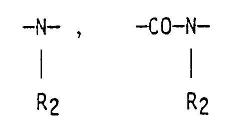 dans ces formules, R₂ représentant un atome d'hydrogène ou un radical alkyle ayant de 1 à 4 atomes de carbone, un radical cyclohexyle ou phényle.

6. Procédé selon l'une des revendications 1 à 5 caractérisé par le fait que le composé aromatique porteur d'au moins un atome d'halogène et d'au moins un groupe hydroxyle répondant à la formule générale (II) est substitué par un ou plusieurs substituants tels que :
. un radical de formule -R₃-OH dans laquelle le radical R₃ représente un lien valentiel ou un radical hydrocarboné divalent, linéaire ou ramifié, saturé ou insaturé ayant de 1 à 4 atomes de carbone, tel que par exemple, méthylène, éthylène, propylène, isopropylène, isopropylidène,
. un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
. un radical alcényle linéaire ou ramifié ayant de 2 à 6 atomes de carbone, de préférence, de 2 à 4 atomes de carbone, tel que vinyle, allyle,
. un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
. un groupe -CHO,
. un groupe acyle ayant de 2 à 6 atomes de carbone,
. un radical de formule -R₃-COOH, R₃ ayant la signification donnée précédemment,
. un radical de formule -R₃-COOR₄ dans laquelle R₃ a la signification donnée précédemment et R₄ représente un radical alkyle, linéaire ou ramifié, ayant de 1 à 6 atomes de carbone,
. un radical de formule -R₃-NH₂ avec un groupe NH₂ N-protégé, R₃ ayant la signification donnée précédemment,
. un radical de formule -R₃-N(R'₄)₂ dans laquelle R₃ a la signification donnée précédemment et les radicaux R'₄ identiques ou différents représentent un atome d'hydrogène ou un radical alkyle, linéaire ou ramifié, ayant de 1 à 6 atomes de carbone,
. un radical de formule -R₃-CO-N(R'₄)₂ R₃ et R'₄ ayant la signification donnée précédemment,
. un radical de formule -R₃-Z dans laquelle R₃ a la signification donnée précédemment et Z symbolise un atome d'halogène X, tel que précédemment défini, un atome de fluor ou un groupe CF₃.

7. Procédé selon l'une des revendications 1 à 6 caractérisé par le fait que le composé aromatique porteur d'au moins un atome d'halogène et d'au moins un groupe hydroxyle est choisi parmi :
- le bromo-2 phénol
- le bromo-3 phénol
- le bromo-4 phénol
- le bromo-1 naphtol-2
- le bromo-6 naphtol-2
- le bromo-2 méthyl-4 phénol
- le bromo-4 diméthyl-2,6 phénol
- le bromo-4 diméthyl-3,5 phénol
- le dibromo-2,6 méthyl-4 phénol
- le bromo-2-p-crésol
- le bromo-2 chloro-4 phénol
- le bromo-4 chloro-2 phénol
- le bromo-4 chloro-6-o-crésol
- les bromofluorophénols
- les bromo bis-phénols
- les bromo isopropylidène-4,4' bis-phénols

8. Procédé selon l'une des revendications 1 à 7 caractérisé par le fait que l'alcoolate de métal alcalin ou alcalino- terreux répond à la formule générale (III) dans laquelle R représente un radical alkyle, alcényle, alcadiényle, alcynyle, linéaire ou ramifié, ayant de préférence moins de 6 atomes de carbone ou un radical cyclo- hexyle ou benzyle.

9. Procédé selon l'une des revendications 1 à 8 caractérisé par le fait que l'alcoolate de métal alcalin est un alcoolate de sodium ou de potassium d'un alcanol primaire ou secondaire ayant de 1 à 4 atomes de carbone, de préférence, le méthylate de sodium ou l'éthylate de sodium.

10. Procédé selon la revendicaton 1 caractérisé par le fait que le composé aromatique polyalkoxylé répond à la formule générale (Ia) : 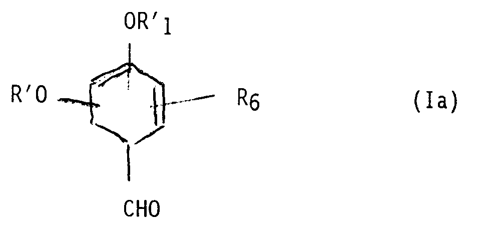 dans laquelle :
- R', R'₁, R₆ identiques ou différents représentent un radical alkyle, linéaire ou ramifié, ayant de 1 à 4 atomes de carbone,
- R₆ pouvant représenter également un atome d'hydrogène, un radical alkoxy, linéaire ou ramifié ayant de 1 à 4 atomes de carbone.

11. Procédé selon la revendication 10 caractérisé par le fait que l'on effectue les étapes suivantes :
1° - On fait réagir un composé aromatique de formule (II) qui est un halogéno hydroxy benzaldéhyde répondant à la formule générale (IIa) : 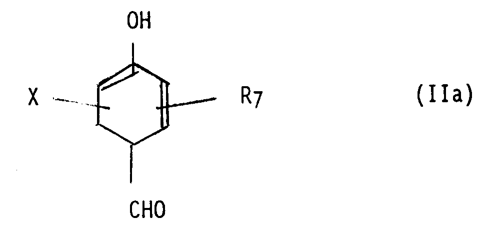 dans laquelle :
- X représente un atome d'iode, de brome ou de chlore,
- R₇ représente un atome d'hydrogène, un atome d'iode, de brome, un atome de chlore, un radical alkyle ayant de 1 à 4 atomes de carbone, un radical alkoxy ayant de 1 à 4 atomes de carbone, un radical hydroxyle,
dans ladite formule (IIa), le radical hydroxyle pouvant être en position ortho-, méta- ou para- de la fonction aldéhyde,
avec un alcoolate de métal alcalin ou alcalino-terreux ayant de 1 à 4 atomes de carbone, en présence d'un catalyseur au cuivre et, éventuellement d'un co-catalyseur,
2° - En fin de réaction d'alkoxylation, on introduit du dioxyde de carbone en une quantité correspondant à la quantité l'alcoolate de métal alcalin ou alcalino-terreux qui n'a pas réagi.
3° - On réalise la O-alkylation du produit obtenu précédemment après alkoxylation, par addition dans le milieu réactionnel organique, d'un agent d'alkylation apportant le reste alkyle R'₁ ayant de 1 à 4 atomes de carbone.

12. Procédé selon l'une des revendications 10 et 11 caractérisé par le fait que le composé aromatique porteur d'au moins un atome d'halogène et d'au moins un groupe hydroxyle répond à la formule générale (IIa) dans laquelle :
. Groupe I :
- le radical OH est en position para par rapport à la fonction CHO,
- l'atome d'halogène X est en position ortho par rapport au radical OH,
- le radical R₇ est en position ortho par rapport au radical OH.
. Groupe II :
- le radical OH est en position ortho par rapport à la fonction CHO,
- l'atome d'halogène X est en position para par rapport au radical OH,
- le radical R₇ est en position ortho par rapport au radical OH.
. Groupe III :
- le radical OH est en position ortho par rapport à la fonction CHO,
- l'atome d'halogène X est en position ortho par rapport au radical OH,
- le radical R₇ est en position para par rapport au radical OH,
. Groupe IV :
- le radical OH est en position méta par rapport à la fonction CHO,
- l'atome d'halogène X est en position ortho par rapport au radical OH,
- le radical R₇ est en position para par rapport au radical OH,
. Groupe V :
- le radical OH est en position méta par rapport à la fonction CHO,
- l'atome d'halogène X est en position para par rapport au radical OH,
- le radical R₇ est en position ortho par rapport au radical OH.

13. Procédé selon l'une des revendications 10 à 12 caractérisé par le fait que le composé aromatique porteur d'au moins un atome d'halogène et d'au moins un groupe hydroxyle répond à l'une des formules suivantes : 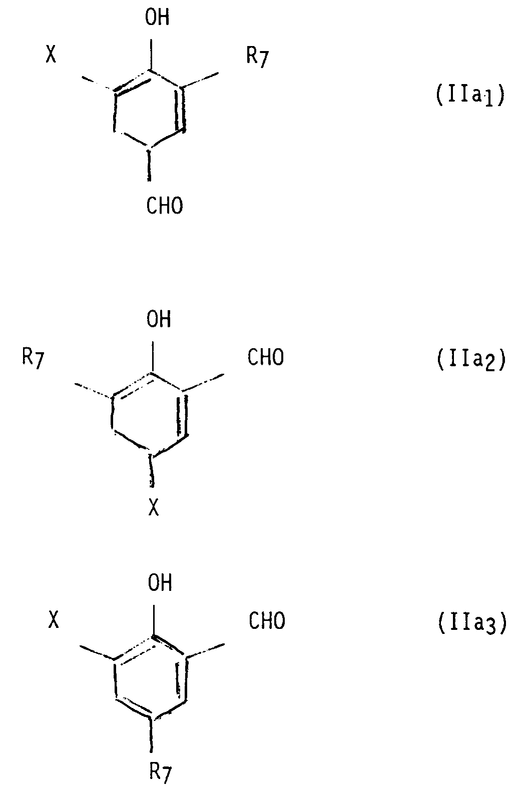 dans lesquelles :
- X et R₇ ont les significations données précédemment.

14. Procédé selon l'une des revendication 10 à 13 caractérisé par le fait que le composé aromatique porteur d'au moins un atome d'halogène et d'au moins un groupe hydroxyle est choisi parmi :
- le bromo-3 hydroxy-4 benzaldéhyde,
- le iodo-3 hydroxy-4 benzaldéhyde
- le dibromo-3,5 hydroxy-4 benzaldéhyde
- le diiodo-3,5 hydroxy-4 benzaldéhyde
- le bromo-5 méthoxy-3 hydroxy-4 benzaldéhyde
- le iodo-5 méthoxy-3 hydroxy-4 benzaldéhyde
- le bromo-5 éthoxy-3 hydroxy-4 benzaldéhyde
- le iodo-5 éthoxy-3 hydroxy-4 benzaldéhyde
- le bromo-3 dihydroxy-4,5 benzaldéhyde
- le iodo-3 dihydroxy-4,5 benzaldéhyde
- le bromo-3 dihydroxy-2,5 benzaldéhyde
- le iodo-3 dihydroxy-2,5 benzaldéhyde
- le bromo-2 hydroxy-4 benzaldéhyde
- le iodo-2 hydroxy-4 benzaldéhyde
- le bromo-4 hydroxy-3 benzaldéhyde
- le iodo-4 hydroxy-3 benzaldéhyde
- le bromo-3 hydroxy-2 benzaldéhyde
- le iodo-3 hydroxy-2 benzaldéhyde
- le bromo-5 hydroxy-2 benzaldéhyde
- le iodo-5 hydroxy-2 benzaldéhyde

15. Procédé selon l'une des revendications 10 à 14 caractérisé par le fait que l'alcoolate de métal alcalin est un alcoolate de sodium ou de potassium d'un alcanol primaire ou secondaire ayant de 1 à 4 atomes de carbone, de préférence, le méthylate de sodium ou l'éthylate de sodium.

16. Procédé selon l'une des revendications 1 à 15 caractérisé par le fait que le catalyseur au cuivre est le cuivre de métal ou un composé du cuivre qui est un composé organique ou inorganique de cuivre I ou de cuivre II choisi parmi le chlorure cuivreux, le chlorure cuivrique, le carbonate de cuivre II basique, le nitrate cuivreux, le nitrate cuivrique, le sulfate cuivrique, l'acétate cuivrique, le trifluorométhylsulfonate cuivrique, l'hydroxyde cuivrique, le picolinate de cuivre II, le méthylate de cuivre I, le méthylate de cuivre II, le chélate de cuivre II de la 8-quinoléine, les composés de cuivre de formule ClCuOCH₃, Cu₂(OCH₃)₂(acac)₂.

17. Procédé selon l'une des revendications 1 à 16 caractérisé par le fait que l'on ajoute un co-catalyseur choisi parmi les carbonates organiques, les carbonates mixtes organo-métalliques, le dioxyde de carbone ou les composés susceptibles de former du dioxyde de carbone.

18. Procédé selon l'une des revendications 1 à 16 caractérisé par le fait que l'agent alkylation dans la réaction de O-alkylation est :
- le diméthylsulfate ou le diméthylcarbonate,
- un halogénure d'alkyle inférieur répondant à la formule générale (V) :
R_{1-X} (V)
dans ladite formule, X représente un atome de brome, de chlore ou d'iode et R₁ représente un radical alkyle linéaire ou ramifié ayant de 1 à 6 atomes de carbone, de préférence, le chlorure de méthyle, le chloroéthane, le bromure de méthyle et le bromoéthane.

19. Procédé selon l'une des revendications 1 à 18 caractérisé par le fait que la réaction est conduite dans un solvant constitué par l'alcanol correspondant à l'alcoolate de métal alcalin ou alcalino-terreux utilisé.

20. Procédé selon l'une des revendications 1 à 19 caractérisé par le fait que la quantité d'alcoolate de métal alcalin ou alcalino-terreux utilisée est supérieure à la quantité stoechiométrique nécessaire pour transformer le composé de formule (II) en phénate alcalin ou alcalino-terreux correspondant et pour transformer le ou les atomes d'halogène qu'il comporte en groupements alkoxy.

21. Procédé selon l'une des revendications 1 à 20 caractérisé par le fait que l'alcoolate de métal alcalin ou alcalino-terreux est mis en oeuvre en une quantité correspondant à la quantité stoechiométrique nécessaire pour salifier le ou les groupes hydroxyle plus la quantité égale de 1, 1 à 5 fois, et de préférence de 1, 5 fois à 3 fois, la quantité stoechiométrique nécessaire pour transformer le ou les atomes d'halogène en groupements alkoxy.

22. Procédé selon l'une des revendications 1 à 21 caractérisé par le fait que la quantité de dioxyde de carbone exprimée par rapport à l'alcoolate de métal alcalin ou alcalino-terreux, introduite en fin de réaction d'alkoxylation, est égale à la quantité stoechiométrique jusqu'à un excès pouvant atteindre 50 % mais, de préférence, compris entre 10 et 20 %.

23. Procédé selon l'une des revendications 1 à 22 caractérisé par le fait que l'on introduit avant l'agent d'alkylation, un sel sous forme d'iodure de métal alcalin, de préférence de sodium.

24. Procédé selon l'une des revendications 1 à 23 caractérisé par le fait que la quantité d'agent d'alkylation engagée est au moins égale à la quantité d'alcoolate de métal alcalin ou alcalino-terreux ou en excès pouvant atteindre 100%.

25. Procédé selon l'une des revendications 1 à 24 caractérisé par le fait que la quantité d'agent d'alkylation engagé est égale ou en excès pouvant atteindre 100 % par rapport à la stoechiométrie de la réaction de O-alkylation.

26. Procédé selon l'une des revendications 1 à 25 caractérisé par le fait que l'on introduit le dioxyde de carbone, en fin de réaction d'alkoxylation, dans le milieu réactionnel maintenu à une température d'alkoxylation comprise entre 60°C et 220°C, et de préférence entre 100°C et 180°C ou bien amené à une température inférieure de 10°C à 30°C à la température d'alkoxylation.

## Claims

1. A process for the preparation of a polyalkoxylated aromatic compound from an aromatic compound which carries at least one halogen atom and at least one hydroxyl group, comprising carrying out:
- an alkoxylation reaction by reacting said compound with an excess of alkali metal or alkaline earth metal alcoholate, in the presence of copper and/or a copper compound, possibly in the presence of a co-catalyst,
- an O-alkylation reaction by reacting the aromatic compound thus obtained which carries at least one alkoxy group and at least one hydroxyl group with an alkylating agent selected from lower alkyl halides, dialkylsulphates, dialkylcarbonates, and which is characterised by the fact that at the end of the alkoxylation reaction carbon dioxide is introduced in a quantity corresponding to the quantity of unreacted alkali metal or alkaline earth metal alcoholate.

2. A process according to Claim 1, characterised by the fact that the excess of alkali metal or alkaline earth metal alcoholate is converted into dialkylcarbonate which is used as an alkylating agent by reacting it at the end of the alkoxylation reaction with carbon dioxide, then with a sufficient quantity of alkyl halide.

3. A process according to one of Claims 1 or 2, characterised by the fact that the polyalkoxylated aromatic compound corresponds to general formula (I): 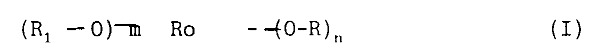 wherein:
- m and n are integers greater than or equal to 1, m + n preferably being between 2 and 6,
- Rₒ represents an aromatic cyclic radical with at least 5 atoms in the cycle, possibly substituted, and representing at least one of the following radicals:
. a monocyclic or polycyclic aromatic carbocyclic radical,
. a monocyclic or polycyclic aromatic heterocyclic radical, comprising at least one of the heteroatoms O, N and S,
- R represents a hydrocarbon radical with 1 to 12 carbon atoms which can be a straight or branched, saturated or unsaturated acyclic aliphatic radical; a monocyclic or polycyclic, saturated or unsaturated cycloaliphatic radical; a straight or branched, saturated or unsaturated, cycloaliphatic- or arylaliphatic radical,
- R₁ is a straight or branched alkyl radical with 1 to 6 carbon atoms.

4. A process according to one of Claims 1 to 3, characterised by the fact that the following steps are carried out:
i) an aromatic compound which carries at least one halogen atom and at least one hydroxyl group corresponding to general formula (II): 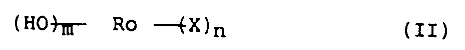 wherein :
- m and n are integers greater than or equal to 1, with m + n preferably being between 2 and 6,
- X represents an iodine, bromine or chlorine atom,
- Rₒ represents an aromatic cyclic radical with at least 5 atoms in the cycle, possibly substituted, and representing at least one of the following radicals:
. a monocyclic or polycyclic aromatic carbocyclic radical,
. a monocyclic or polycyclic aromatic heterocyclic radical, comprising at least one of the heteroatoms O, N and S,
is reacted with an alkali metal or an alkaline earth metal alcoholate of general formula (III) :
M^{w+} [ O - R ]_{w}- (III)
wherein:
- M represents an alkali metal or an alkaline earth metal,
- w represents the valency of the alkali metal or alkaline earth metal,
- R represents a hydrocarbon radical with 1 to 12 carbon atoms which can be a straight or branched, saturated or unsaturated acyclic aliphatic radical; a monocyclic or polycyclic, saturated or unsaturated, cycloaliphatic radical; a straight or branched, saturated or unsaturated cycloaliphatic- or arylaliphatic radical.
in the presence of a catalyst with copper and possibly a co-catalyst.
ii) Carbon dioxide is introduced at the end of the alkoxylation reaction,
iii) O-alkylation takes place of the product previously obtained after alkoxylation by adding to the organic reaction medium an alkylating agent selected from lower alkyl halides, dialkylsulphates and dialkylcarbonates.

5. A process according to one of Claims 1 to 4, characterised by the fact that the aromatic compound which carries at least one halogen atom and at least one hydroxyl group corresponds to general formula (II) in which the radical Ro symbolises:
i) - a monocyclic or polycyclic aromatic carbocyclic radical,
ii) - a monocyclic or polycyclic aromatic heterocyclic radical,
iii) - a divalent radical constituted by a linkage of groups as defined under paragraphs i) and/or ii), interconnected:
. by a valency bond,
. by an alkylene or alkylidene radical with 1 to 4 carbon atoms, preferably a methylene or isopropylidene radical,
. by one of the following groups:
-O- , -CO- ,
-S- , -SO- , -SO₂- , 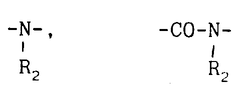 in which formulae R₂ represents a hydrogen atom or an alkyl radical with 1 to 4 carbon atoms, a cyclohexyl or phenyl radical.

6. A process according to one of Claims 1 to 5, characterised by the fact that the aromatic compound which carries at least one halogen atom and at least one hydroxyl group corresponding to the general formula (II) is substituted by one or more substituents such as:
. a radical of the formula -R₃-OH in which the R₃ radical represents a valency bond or a saturated or unsaturated, straight or branched divalent hydrocarbon radical with 1 to 4 carbon atoms such as methylene, ethylene, propylene, isopropylene, isopropylidene,
. a straight or branched alkyl radical with 1 to 6 carbon atoms, preferably 1 to 4 carbon atoms such as methyl, ethyl, propyl, isopropyl, butyl, isobutyl, sec-butyl, or tert-butyl,
. a straight or branched alkenyl radical with 2 - 6 carbon atoms, preferably 2 to 4 carbon atoms, such as vinyl, allyl,
. a straight or branched alkoxy radical with 1 to 6 carbon atoms, preferably 1 to 4 carbon atoms such as a methoxy, ethoxy, propoxy, isopropoxy or butoxy radical
. a -CHO group,
. an acyl group with 2 to 6 carbon atoms,
. a radical of the formula -R₃-COOH, R₃ having the meaning given above,
. a radical of the formula -R₃-COOR₄ in which R₃ has the meaning given above and R₄ represents a straight or branched alkyl radical with 1 to 6 carbon atoms,
. a radical of the formula -R₃-NH₂ with a protected NH₂ N group, R₃ having the meaning given above,
. radical of the formula -R₃-N(R'₄)₂ in which R₃ has the meaning given above and the radicals R'₄ which may be the same or different represent a hydrogen atom or a straight or branched alkyl radical with 1 to 6 carbon atoms,
. a radical of the formula -R₃-CO-N(R'₄)₂, R₃ and R₄ having the meaning given above,
. a radical of the formula -R₃-Z in which R₃ has the meaning given above, and Z symbolises a halogen atom X as defined above, a fluorine atom or a CF₃ group.

7. A process according to one of Claims 1 to 6, characterised by the fact that the aromatic compound which carries at least one halogen atom and at least one hydroxyl group is selected from:
- 2-bromophenol
- 3-bromophenol
- 4-bromophenol
- 1-bromo-2-naphthol
- 6-bromo-2-naphthol
- 2-bromo-4-methyl phenol
- 4-bromo-2,6-dimethyl phenol
- 4-bromo-3,5-dimethyl phenol
- 2.6-dibromo-4-methyl phenol
- 2-p-bromocresol
- 2-bromo-4-chlorophenol
- 4-bromo-2-chlorophenol
- 4-bromo-6-o-chlorocresol
- bromofluorophenols
- bromo bis-phenols
- bromo-4,4'-isopropylidene bis-phenols.

8. A process according to one of Claims 1 to 7, characterised by the fact that the alkali metal or alkaline earth metal alcoholate corresponds to the general formula (III) in which R represents a straight or branched alkyl, alkenyl, alkadienyl or alkynyl radical with preferably less than 6 carbon atoms or a cyclo- hexyl or benzyl radical.

9. A process according to one of Claims 1 to 8, characterised by the fact that the alkali metal alcoholate is a sodium alcoholate or potassium alcoholate of a primary or secondary alkanol with 1 to 4 carbon atoms, preferably sodium methylate or sodium ethylate.

10. A process according to Claim 1, characterised by the fact that the polyalkoxylated aromatic compound corresponds to the general formula (Ia): 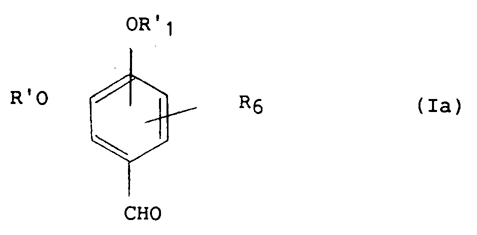 in which:
- R', R'₁, R₆ which are the same or different represent a straight or branched alkyl radical with 1 to 4 carbon atoms,
- R₆ also being able to represent a hydrogen atom, a straight or branched alkoxy radical with 1 to 4 carbon atoms.

11. A process according to Claim 10, characterised by the fact that the following steps are carried out:
i) Reacting an aromatic compound of formula (II) which is a halohydroxy benzaldehyde corresponding to general formula (IIa): 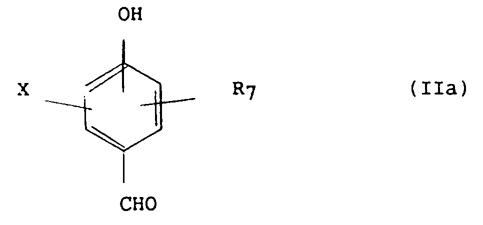 in which:
- X represents an iodine, bromine or chlorine atom
- R₇ represents a hydrogen atom, an iodine atom, bromine atom, chlorine atom, an alkyl radical with 1 to 4 carbon atoms, an alkoxy radical with 1 to 4 carbon atoms, a hydroxyl radical, in which formula (IIa), the hydroxyl radical can be in the ortho-, meta- or para- position of the aldehyde function,
with an alkali metal or alkaline earth metal alcoholate with 1 to 4 carbon atoms in the presence of a catalyst with copper and possibly in the presence of a co-catalyst,
ii) at the end of the alkoxylation reaction, carbon dioxide is introduced in a quantity corresponding to the quantity of unreacted alkali metal or alkaline earth metal alcoholate.
iii) O-alkylation is carried out of the product previously obtained after alkoxylation, by adding to the organic reaction medium an alkylating agent which provides the alkyl residue R'1 with 1 to 4 carbon atoms.

12. A process according to one of Claims 10 and 11, characterised by the fact that the aromatic compound which carries at least one halogen atom and at least one hydroxyl group corresponds to the general formula (IIa), in which:
Group I:
- the OH radical is in the para position relative to the CHO function,
- the halogen atom X is in the ortho position relative to the OH radical,
- the R₇ radical is in the ortho position relative to the OH radical.
Group II:
- the OH radical is in the ortho position relative to the CHO function,
- the halogen atom X is in the para position relative to the OH radical,
- the R₇ radical is in the ortho position relative to the OH radical.
Group III:
- the OH radical is in the ortho position relative to the CHO function,
- the halogen atom X is in the ortho position relative to the OH radical,
- the R₇ radical is in the para position relative to the OH radical,
Group IV:
- the OH radical is in the meta position relative to the CHO function,
- the halogen atom X is in the ortho position relative to the OH radical,
- the R₇ radical is in the para position relative to the OH radical
Group V:
- the OH radical is in the meta position relative to the CHO function,
- the halogen atom X is in the para position relative to the OH radical,
- the R₇ radical is in the ortho position relative to the OH radical.

13. A process according to one of Claims 10 to 12, characterised by the fact that the aromatic compound which carries at least one halogen atom and at least one hydroxyl group corresponds to one of the following formulae: 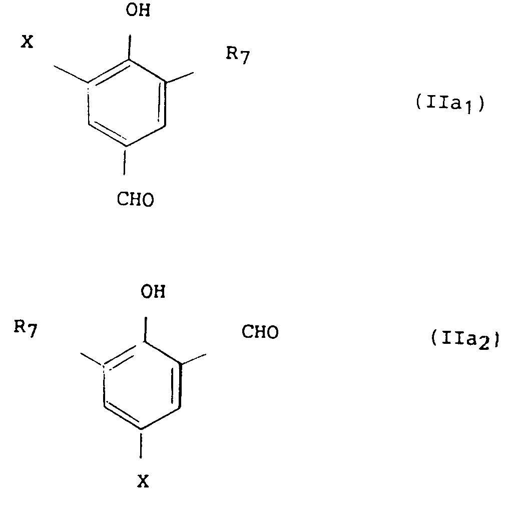 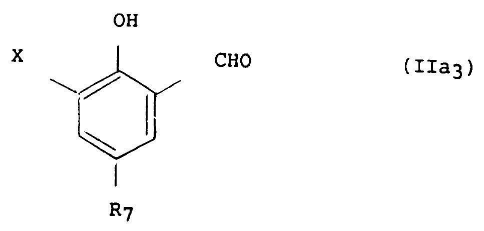 wherein:
X and R₇ have the meanings given above.

14. A process according to one of Claims 10 to 13, characterised by the fact that the aromatic compound carrying at least one halogen atom and at least one hydroxyl group is selected from:
- 3-bromo 4-hydroxy benzaldehyde,
- 3-iodo 4-hydroxy benzaldehyde
- 3,5-dibromo 4-hydroxy benzaldehyde
- 3,5 diiodo 4-hydroxy benzaldehyde
- 5-bromo 3-methoxy 4-hydroxy benzaldehyde
- 5-iodo 3-methoxy 4-hydroxy benzaldehyde
- 5-bromo 3-ethoxy 4-hydroxy benzaldehyde
- 5-iodo 3-ethoxy 4-hydroxy benzaldehyde
- 3-bromo 4,5-dihydroxy benzaldehyde
- 3-iodo 4,5-dihydroxy benzaldehyde
- 3-bromo 2,5-dihydroxy benzaldehyde
- 3-iodo 2,5-dihydroxy benzaldehyde
- 2-bromo 4-hydroxy benzaldehyde
- 2-iodo 4-hydroxy benzaldehyde
- 4-bromo 3-hydroxy benzaldehyde
- 4-iodo 3-hydroxy benzaldehyde
- 3-bromo 2-hydroxy benzaldehyde
- 3-iodo 2-hydroxy benzaldehyde
- 5-bromo 2-hydroxy benzaldehyde
- 5-iodo 2-hydroxy benzaldehyde

15. A process according to one of Claims 10 to 14, characterised by the fact that the alkali metal alcoholate is a sodium or potassium alcoholate of a primary or secondary alkanol with 1 to 4 carbon atoms, preferably sodium methylate or sodium ethylate.

16. A process according to one of Claims 1 to 15, characterised by the fact that the catalyst with copper is metallic copper or a copper compound which is an organic or inorganic compound of copper I or copper II, selected from cuprous chloride, cupric chloride, basic copper II carbonate, cuprous nitrate, cupric nitrate, cupric sulphate, cupric acetate, cupric trifluoromethyl sulphonate, cupric hydroxide, copper II picolinate, copper I methylate, copper II methylate, copper II chelate of 8-quinoline, or copper compounds of the formula ClCuOCH₃, Cu₂(OCH₃)₂(acac)₂.

17. A process according to one of Claims 1 to 16, characterised by the fact that a co-catalyst selected from organic carbonates, combined organo-metallic carbonates, carbon dioxide or compounds capable of forming carbon dioxide is added.

18. A process according to one of Claims 1 to 16, characterised by the fact that the alkylating agent in the O-alkylation reaction is:
- dimethylsulphate or dimethylcarbonate,
- a lower alkyl halide corresponding to general formula (V):
R₁₋ₓ (V)
in which formula, X represents a bromine, chlorine or iodine atom and R₁ represents a straight or branched alkyl radical with 1 to 6 carbon atoms, preferably methyl chloride, chloroethane, methyl bromide and bromoethane.

19. A process according to one of Claims 1 to 18, characterised by the fact that the reaction is carried out in a solvent constituted by the alkanol corresponding to the alkali metal or alkaline earth metal alcoholate used.

20. A process according to one of Claims 1 to 19, characterised by the fact that the quantity of alkali metal or alkaline earth metal alcoholate used is greater than the stoichiometric quantity required to convert the formula (II) compound into the corresponding alkali metal or alkaline earth metal phenate and to convert the halogen atom(s) which it comprises into alkoxy groups.

21. A process according to one of Claims 1 to 20, characterised by the fact that the alkali metal or alkaline earth metal alcoholate is used in a quantity corresponding to the stoichiometric quantity required to salify the hydroxyl group(s) plus a quantity equal to 1.1 to 5 times, and preferably 1.5 to 3 times, the stoichiometric quantity required to convert the halogen atom(s) to alkoxy groups.

22. A process according to one of Claims 1 to 21, characterised by the fact that the quantity of carbon dioxide, expressed in relation to the alkali metal or alkaline earth metal alcoholate introduced at the end of the alkoxylation reaction, is equal to the stoichiometric quantity up to an excess going to a maximum of 50%, but preferably from 10 to 20%.

23. A process according to one of Claims 1 to 22, characterised by the fact that a salt in the form of alkali metal iodide, preferably sodium iodide, is introduced before the alkylating agent.

24. A process according to one of Claims 1 to 23, characterised by the fact that the quantity of alkylating agent involved is at least equal to the quantity of alkali metal or alkaline earth metal alcoholate, or in excess by up to 100%.

25. A process according to one of Claims 1 to 24, characterised by the fact that the quantity of alkylating agent used is equal to or in excess by up to 100% relative to the stoichiometry of the O-alkylation reaction.

26. A process according to one of Claims 1 to 25, characterised by the fact that the carbon dioxide is introduced at the end of the alkoxylation reaction into the reaction medium which is kept at an alkoxylating temperature of between 60°C and 220°C, and preferably between 100°C and 180°C, or brought to a temperature of 10 to 30°C below the alkoxylating temperature.

## Patentansprüche

1. Verfahren zur Herstellung einer polyalkoxylierten aromatischen Verbindung, ausgehend von einer aromatischen Verbindung, die mindestens ein Halogenatom und mindestens eine Hydroxylgruppe enthält, bestehend aus:
- einer Alkoxylierungsreaktion, bei der man diese Verbindung mit einem Überschuß an Alkali- oder Erdalkalialkoholat in Gegenwart von Kupfer und/oder einer Kupferverbindung, gegebenenfalls in Gegenwart eines Co-Katalysators reagieren läßt,
- eine O-Alkylierungsreaktion, bei der man die so erhaltene aromatische Verbindung, die mindestens eine Alkoxygruppe und mindestens eine Hydroxylgruppe enthält, mit einem Alkylierungsmittel, ausgewählt aus den niederen Alkylhalogeniden, Dialkylsulfaten und Dialkylcarbonaten, reagieren läßt, dadurch gekennzeichnet, daß man am Schluß der Alkoxylierungsreaktion Kohlendioxid in einer Menge hinzugibt, die der nicht umgesetzten Alkali- oder Erdalkalialkoholatmenge entspricht.

2. Verfahren nach Anspruch 1, dadurch gekennzeichnet, daß man den Überschuß an Alkali- oder Erdalkalialkoholat in ein Dialkylcarbonat überführt, was als Alkylierungsmittel verwendet wird, indem man es am Ende der Alkoxylierungsreaktion mit Kohlendioxid, und dann mit dem eingesetzten Alkylhalogenid in ausreichender Menge reagieren läßt.

3. Verfahren nach einem der Ansprüche 1 und 2, dadurch gekennzeichnet, daß die polyalkoxylierte aromatische Verbindung der allgemeinen Formel (I): 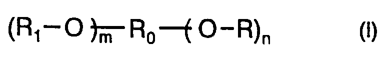 entspricht, in der
- m und n ganze Zahlen größer oder gleich 1 sind, vorzugsweise ist m + n zwischen 2 und 6,
- R₀ einen aromatischen cyclischen Rest mit mindestens 5 Kohlenstoffatomen im Ring, die gegebenenfalls substituiert sind, darstellt, und mindestens einen der folgenden Reste darstellt:
· einen monocyclischen oder polycyclischen aromatischen carbocyclischen Rest,
· einen monocyclischen oder polycyclischen aromatischen heterocyclischen Rest, der mindestens eins der Heteroatome O, N und S enthält,
- R einen Kohlenwasserstoffrest mit 1 bis 12 Kohlenstoffatomen darstellt, der ein gesättigter oder ungesättigter, linearer oder verzweigter acyclischer aliphatischer Rest; ein gesättigter oder ungesättigter, monocyclischer oder polycyclischer cycloaliphatischer Rest; ein gesättigter oder ungesättigter, linearer oder verzweigter arylaliphatischer oder cycloaliphatischer Rest sein kann,
- R₁ ein linearer oder verzweigter Alkylrest mit 1 bis 6 Kohlenstoffatomen ist.

4. Verfahren nach einem der Ansprüche 1 bis 3, dadurch gekennzeichnet, daß man es in folgenden Schritten durchführt:
1) Man läßt die aromatische Verbindung, die mindestens ein Halogenatom und mindestens eine Hydroxylgruppe enthält, und der allgemeinen Formel (II): 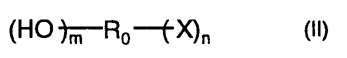 entspricht, in der:
- m und n ganze Zahlen größer oder gleich 1 sind, vorzugsweise ist m + n zwischen 2 und 6,
- X ein Iod-, Brom- oder Chloratom darstellt,
- R₀ einen aromatischen cyclischen Rest mit mindestens 5 Kohlenstoffatomen in dem Ring darstellt, die gegebenenfalls substituiert sind, und mindestens einen der folgenden Reste darstellt:
· einen monocyclischen oder polycyclischen aromatischen carbocyclischen Rest,
· einen monocyclischen oder polycyclischen aromatischen heterocyclischen Rest, der mindestens eines der Heteroatome O, N und S enthält,
- und mindestens ein Alkali- oder Erdalkalialkoholat der allgemeinen Formel (III):
M^{w+} [ O-R ]_{w-} (III)
in der:
- M ein Alkali- oder Erdalkalimetall darstellt,
- w die Wertigkeit des Alkali- oder Erdalkalimetalls darstellt,
- R einen Kohlenwasserstoffrest mit 1 bis 12 Kohlenstoffatomen darstellt, der ein gesättigter oder ungesättigter, linearer oder verzweigter acyclischer aliphatischer Rest; ein gesättigter oder ungesättigter, monocyclischer oder polycyclischer cycloaliphatischer Rest; ein gesättigter oder ungesättigter, linearer oder verzweigter cycloaliphatischer oder arylaliphatischer Rest sein kann
in Gegenwart eines Kupferkatalysators und gegebenenfalls eines Co-Kata lysators reagieren läßt,
2) am Schluß der Alkoxylierungsreaktion gibt man Kohlendioxid hinzu,
3) man führt die O-Alkylierung des zuvor erhaltenen Produktes nach der Alkoxylierung durch Zugabe eines Alkylierungsmittels in das organische Reaktionsmilieu durch, wobei das Alkylierungsmittel ausgewählt ist aus den niederen Alkylhalogeniden, Dialkylsulfaten und Dialkylcarbonaten.

5. Verfahren nach einem der Ansprüche 1 bis 4, dadurch gekennzeichnet, daß die aromatische Verbindung, die mindestens ein Halogenatom und mindestens eine Hydroxylgruppe enthält, der allgemeinen Formel (II) entspricht, in der der Rest R₀
1) einen monocyclischen oder polycyclischen aromatischen carbocyclischen Rest,
2) einen monocyclischen oder polycyclischen aromatischen heterocyclischen Rest,
3) einen divalenten Rest, der aus einer Verkettung der Gruppen gebildet wird, wie sie in dem Abschnitt 1 und/oder 2 definiert sind, wobei diese miteinander verbunden werden:
· durch eine Valenzbindung,
· durch einen Alkylen- oder Alkylidenrest mit 1 bis 4 Kohlenstoffatomen, vorzugsweise einen Methylen- oder Isopropylidenrest,
· durch eine der folgenden Gruppen:
-O- , -CO- ,
-S- , -SO- , -SO₂- , 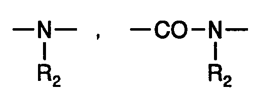 wobei in diesen Formeln R₂ ein Wasserstoffatom oder einen Alkylrest mit 1 bis 4 Kohlenstoffatomen, einen Cyclohexyl- oder einen Phenylrest darstellt,
darstellt.

6. Verfahren nach einem der Ansprüche 1 bis 5, dadurch gekennzeichnet, daß die der allgemeinen Formel (II) entsprechende aromatische Verbindung, die mindestens ein Halogenatom und mindestens eine Hydroxylgruppe enthält, durch einen oder mehrere der folgenden Substituenten substituiert ist:
· einen Rest der Formel -R₃-OH, in der der Rest R₃ eine Valenzbindung oder einen linearen oder verzweigten, gesättigten oder ungesättigten divalenten Kohlenwasserstoffrest mit 1 bis 4 Kohlenstoffatomen, wie z.B. Methylen, Ethylen, Propylen, Isopropylen oder Isopropyliden, darstellt,
· einen linearen oder verzweigten Alkylrest mit 1 bis 6 Kohlenstoffatomen, vorzugsweise 1 bis 4 Kohlenstoffatomen, wie z.B. Methyl, Ethyl, Propyl, Isopropyl, Butyl, Isobutyl, sek.-Butyl oder tert.-Butyl,
· einen linearen oder verzweigten Alkenylrest mit 2 bis 6 Kohlenstoffatomen, vorzugsweise mit 2 bis 4 Kohlenstoffatomen, wie z.B. Vinyl, Allyl,
· einen linearen oder verzweigten Alkoxyrest mit 1 bis 6 Kohlenstoffatomen, vorzugsweise mit 1 bis 4 Kohlenstoffatomen, wie z.B. Methoxy-, Ethoxy-, Propoxy-, Isopropoxy- oder Butoxyrest,
· eine CHO-Gruppe,
· eine Acylgruppe mit 2 bis 6 Kohlenstoffatomen,
· einen Rest der Formel -R₃-COOH, R₃ hat die gleiche Bedeutung wie im vorangegangenen,
· einen Rest der Formel -R₃-COOR₄, in der R₃ die gleiche Bedeutung wie im vorangegangenen hat und R₄ ein linearer oder verzweigter Alkylrest mit 1 bis 6 Kohlenstoffatomen ist,
· einen Rest der Formel -R₃-NH₂ mit einer N-geschützten NH₂-Gruppe, R₃ hat die gleiche Bedeutung wie im vorangegangenen,
· einen Rest der Formel -R₃-N(R'₄)₂, in der R₃ die gleiche Bedeutung wie im vorangegangenen hat und die Reste R'₄, identisch oder verschieden, ein Wasserstoffatom oder ein linearer oder verzweigter Alkylrest mit 1 bis 6 Kohlenstoffatomen sind,
· einen Rest der Formel -R₃-CO-N(R'₄)₂, R₃ und R'₄ haben die gleiche Bedeutung wie im vorangegangenen,
· einen Rest der Formel -R₃-Z, in der R₃ die gleiche Bedeutung wie im vorangegangenen hat und Z ein Halogenatom X darstellt, wie z.B. im vorangegangenen definiert, ein Fluoratom oder eine CF₃-Gruppe.

7. Verfahren nach einem der Ansprüche 1 bis 6, dadurch gekennzeichnet, daß die aromatische Verbindung, die mindestens ein Halogenatom und mindestens eine Hydroxylgruppe enthält, ausgewählt ist aus:
- 2-Bromphenol
- 3-Bromphenol
- 4-Bromphenol
- 1-Bromnapht-2-ol
- 6-Bromnapht-2-ol
- 2-Brom-4-methylphenol
- 4-Brom-2,6-dimethylphenol
- 4-Brom-3,5-dimethylphenol
- 2,6-Dibrom-4-methylphenol
- 2-Brom-p-kresol
- 2-Brom-4-chlorphenol
- 4-Brom-2-chlorphenol
- 4-Brom-6-chlor-o-kresol
- Bromfluorphenole
- Brom-bis-phenole
- Brom-4,4'-isopropyliden-bis-phenole

8. Verfahren nach einem der Ansprüche 1 bis 7, dadurch gekennzeichnet, daß das Alkali- oder Erdalkalialkoholat der allgemeinen Formel (III) entspricht, in der R einen linearen oder verzweigten Alkyl-, Alkenyl-, Alkadienyl- oder Alcynylrest mit vorzugsweise mindestens 6 Kohlenstoffatomen oder einen Cyclohexyl- oder Benzylrest darstellt.

9. Verfahren nach einem der Ansprüche 1 bis 8, dadurch gekennzeichnet, daß das Alkalialkoholat ein Natriumalkoholat oder ein Kaliumalkoholat eines primären oder sekundären Alkohols mit 1 bis 4 Kohlenstoffatomen, vorzugsweise Natriummethylat oder Natriumethylat, ist.

10. Verfahren nach Anspruch 1, dadurch gekennzeichnet, daß die polyalkoxylierte aromatische Verbindung der allgemeinen Formel (Ia) entspricht: 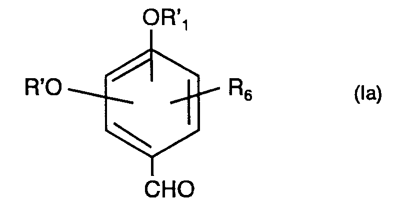 in der:
- R', R'₁ und R₆, identisch oder verschieden, einen linearen oder verzweigten Alkylrest mit 1 bis 4 Kohlenstoffatomen darstellen,
- R₆ gleichermaßen ein Wasserstoffatom oder einen linearen oder verzweigten Alkoxyrest mit 1 bis 4 Kohlenstoffatomen darstellt.

11. Verfahren nach Anspruch 10, dadurch gekennzeichnet, daß man es in folgenden Schritten durchführt:
1) Man läßt eine aromatische Verbindung der Formel (II), die Halogenhydroxybenzaldehyd, entsprechend der allgemeinen Formel (IIa): 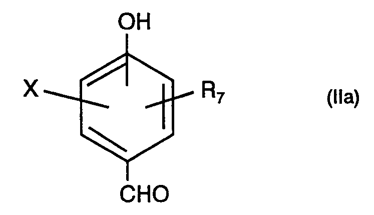 ist, in der:
- X ein Iod-, Brom- oder Chloratom darstellt,
- R₇ ein Wasserstoffatom, ein Iod-, Brom- oder Chloratom, einen Alkylrest mit 1 bis 4 Kohlenstoffatomen, einen Alkoxyrest mit 1 bis 4 Kohlenstoffatomen oder einen Hydroxylrest darstellt, in dieser Formel (IIa) kann der Hydroxylrest in ortho-, meta- oder para-Position zu Aldehydfunktion stehen,
mit einem Alkali- oder Erdalkalialkoholat mit 1 bis 4 Kohlenstoffatomen in Gegenwart eines Kupferkatalysators und gegebenenfalls eines Co-Katalysators reagieren läßt.
2) Am Schluß der Alkoxylierungsreaktion gibt man Kohlendioxid in einer der nicht umgesetzten Alkali- oder Erdalkalialkoholatmenge entsprechenden Menge hinzu.
3) Die O-Alkylierung des zuvor erhaltenen Produktes führt man nach der Alkoxylierung durch Zugabe eines Alkylierungsmittels, das einen Alkylrest R'₁ mit 1 bis 4 Kohlenstoffatomen aufweist, in das organische Reaktionsmilieu durch.

12. Verfahren nach einem der Ansprüche 10 und 11, dadurch gekennzeichnet, daß die aromatische Verbindung, die mindestens ein Halogenatom und mindestens eine Hydroxylgruppe enthält, der allgemeinen Formel (IIa) entspricht, in der:
· Gruppe I:
- der OH-Rest in para-Position zur CHO-Funktion steht,
- das Halogen X-Atom in ortho-Position zum OH-Rest steht,
- der Rest R₇ in ortho-Position zum OH-Rest steht.
· Gruppe II:
- der OH-Rest in ortho-Position zur CHO-Gruppe steht,
- das Halogenatom X in para-Position zum OH-Rest steht,
- der Rest R₇ in ortho-Position zum OH-Rest steht.
· Gruppe III:
- der OH-Rest in ortho-Position zur CHO-Funktion steht,
- das Halogenatom X in ortho-Position zum OH-Rest steht,
- der R₇-Rest in para-Position zum OH-Rest steht.
· Gruppe IV:
- der OH-Rest in meta-Position zur CHO-Funktion steht,
- das Halogenatom X in ortho-Position zum OH-Rest steht,
- der Rest R₇ in para-Position zum OH-Rest steht.
· Gruppe V:
- der OH-Rest in meta-Position zur CHO-Funktion steht,
- das Halogenatom X in para-Position zum OH-Rest steht,
- der Rest R₇ in ortho-Position zum OH-Rest steht.

13. Verfahren nach einem der Ansprüche 10 bis 12, dadurch gekennzeichnet, daß die aromatische Verbindung, die mindestens ein Halogenatom und mindestens eine Hydroxylgruppe enthält, einer der folgenden Formeln entspricht: 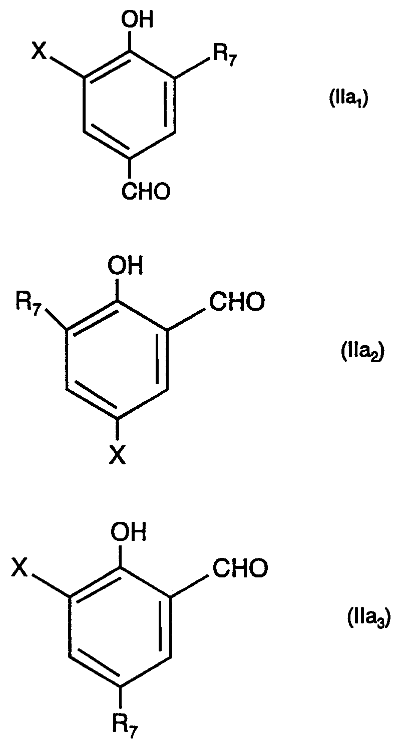 in denen:
- X und R₇ die gleiche Bedeutung haben wie im vorangegangenen.

14. Verfahren nach einem der Ansprüche 10 bis 13, dadurch gekennzeichnet, daß die aromatische Verbindung, die mindestens ein Halogenatom und mindestens eine Hydroxylgruppe enthält, ausgewählt ist aus:
- 3-Brom-4-hydroxybenzaldehyd
- 3-Iod-4-hydroxybenzaldehyd
- 3,5-Dibrom-4-hydroxybenzaldehyd
- 3,5-Diiod-4-hydroxybenzaldehyd
- 5-Brom-3-methoxy-4-hydroxybenzaldehyd
- 5-Iod-3-methoxy-4-hydroxybenzaldehyd
- 5-Brom-3-ethoxy-4-hydroxybenzaldehyd
- 5-Iod-3-ethoxy-4-hydroxybenzaldehyd
- 3-Brom-4,5-dihydroxybenzaldehyd
- 3-Iod-4,5-dihydroxybenzaldehyd
- 3-Brom-2,5-dihydroxybenzaldehyd
- 3-Iod-2,5-dihydroxybenzaldehyd
- 2-Brom-4-hydroxybenzaldehyd
- 2-Iod-4-hydroxybenzaldehyd
- 4-Brom-3-hydroxybenzaldehyd
- 4-Iod-3-hydroxybenzaldehyd
- 3-Brom-2-hydroxybenzaldehyd
- 3-Iod-2-hydroxybenzaldehyd
- 5-Brom-2-hydroxybenzaldehyd
- 5-Iod-2-hydroxybenzaldehyd

15. Verfahren nach einem der Ansprüche 10 bis 14, dadurch gekennzeichnet, daß das Alkalialkoholat ein Natriumalkoholat oder ein Kaliumalkoholat eines primären oder sekundären Alkohols mit 1 bis 4 Kohlenstoffatomen, vorzugsweise Natriummethylat oder Natriumethylat ist.

16. Verfahren nach einem der Ansprüche 1 bis 15, dadurch gekennzeichnet, daß der Kupferkatalysator metallisches Kupfer oder eine Kupferverbindung ist, die eine organische oder anorganische Kupfer(I)- oder Kupfer(II)-Verbindung, ausgewählt aus Kupfer(I)-Chlorid, Kupfer(II)-Chlorid, basisches Kupfer(II)-Carbonat, Kupfer(I)-Nitrat, Kupfer(II)-Nitrat, Kupfer(II)-Sulfat, Kupfer(II)-Acetat, Kupfer(II)-Trifluormethylsulfonat, Kupfer(II)-Hydroxid, Kupfer(II)-Picolinat, Kupfer(I)-Methylat, Kupfer(II)-Methylat, das Chelat von Kupfer(II) und 8-Chinolin und die Kupferverbindungen mit der Formel ClCuOCH₃, Cu₂(OCH₃)₂(acac)₂ ist.

17. Verfahren nach einem der Ansprüche 1 bis 16, dadurch gekennzeichnet, daß man einen Co-Katalysator hinzugibt, der ausgewählt ist aus organischen Carbonaten, gemischten organometallischen Carbonaten, Kohlendioxid oder Verbindungen, die Kohlendioxid bilden können.

18. Verfahren nach einem der Ansprüche 1 bis 16, dadurch gekennzeichnet, daß das Alkylierungsmittel in O-Alkylierungsreaktion
- Dimethylsulfat oder Dimethylcarbonat,
- ein niederes Alkylhalogenid der allgemeinen Formel (V):
R₁-X (V)
in der X ein Brom-, Chlor- oder Iodatom ist und R₁ einen linearen oder verzweigten Alkylrest mit 1 bis 6 Kohlenstoffatomen, vorzugsweise Methylchlorid, Chlorethan, Methylbromid und Bromethan darstellt,
ist.

19. Verfahren nach einem der Ansprüche 1 bis 18, dadurch gekennzeichnet, daß die Reaktion in einem Lösemittel durchgeführt wird, das aus einem dem verwendeten Alkali- oder Erdalkalialkoholat korrespondierenden Alkanol gebildet ist.

20. Verfahren nach einem der Ansprüche 1 bis 19, dadurch gekennzeichnet, daß die Menge des verwendeten Alkali- oder Erdalkalialkoholats über der stöchiometrisch notwendigen Menge liegt, die zur Überführung der Verbindung der Formel (II) zum entsprechenden Alkali- oder Erdalkaliphenat oder zur Überführung des Halogenatoms oder der Halogenatome, das oder die sie enthält, zu Alkoxygruppen, notwendig ist.

21. Verfahren nach einem der Ansprüche 1 bis 20, dadurch gekennzeichnet, daß das Alkali- oder Erdalkalialkoholat in einer Menge verwendet wird, die der zur Versalzung der Hydroxylgruppe(n) stöchiometrisch notwendigen Menge plus der 1,1- bis 5-fachen, vorzugsweise der 1,5-bis 3-fachen, der zur Überführung des Halogenatoms oder der Halogenatome zu den Alkoxygruppen notwendigen stöchiometrischen Menge entspricht.

22. Verfahren nach einem der Ansprüche 1 bis 21, dadurch gekennzeichnet, daß die am Ende der Alkoxylierungsreaktion hinzugegebene Kohlendioxidmenge, berechnet als Verhältnis zum Alkali- oder Erdalkalialkoholat, zwischen der stöchiometrischen Menge und einem Überschuß bis zu 50 %, vorzugsweise zwischen 10 und 20 %, entspricht.

23. Verfahren nach einem der Ansprüche 1 bis 22, dadurch gekennzeichnet, daß man vor den Alkylierungsmitteln ein Salz in Form eines Alkaliiodids, vorzugsweise Natriumiodids, hinzugibt.

24. Verfahren nach einem der Ansprüche 1 bis 23, dadurch gekennzeichnet, daß die Menge an eingesetztem Alkylierungsmittel mindestens der Menge an Alkali- oder Erdalkalialkoholat oder einem Überschuß bis zu 100 % entspricht.

25. Verfahren nach einem der Ansprüche 1 bis 24, dadurch gekennzeichnet, daß das eingesetzte Alkylierungsmittel der Stöchiometrie der O-Alkylierungsreaktion oder einem Überschuß bis zu 100 % entspricht.

26. Verfahren nach einem der Ansprüche 1 bis 25, dadurch gekennzeichnet, daß man das Kohlendioxid am Ende der Alkoxylierungsreaktion in das Reaktionsmilieu hinzugibt, das auf einer Alkoxylierungstemperatur zwischen 60 und 220 °C, vorzugsweise zwischen 100 und 180 °C, oder bei einer Temperatur, die 10 bis 30 °C unterhalb der Alkoxylierungstemperatur liegt, hinzugegeben wird.
